# EUROPEAN PATENT SPECIFICATION

(11) **EP 1 212 358 B1**
(45) Date of publication and mention of the grant of the patent: **15.12.2004**
(21) Application number: 00959292.4
(22) Date of filing: 21.08.2000
(51) Int. Cl.: C07K 14/025, C12N 15/861, C12N 5/10

(54) **SYNTHETIC PAPILLOMAVIRUS GENES OPTIMIZED FOR EXPRESSION IN HUMAN CELLS**
SYNTHETISCHE PAPILLOMAVIRUS GENE, WELCHE FÜR EXPRESSION IN MENSCHLICHEN ZELLEN OPTIMIERT SIND
GENES SYNTHETIQUES HUMAINS DU VIRUS DE PAPILLOME

(30) Priority: 25.08.1999 US 150728 P; 07.06.2000 US 210143 P
(43) Date of publication of application: 12.06.2002
(73) Proprietor: Merck & Co., Inc., Rahway, New Jersey 07065-0907 (US)
(72) Inventor: NEEPER, Michael, P., Rahway, NJ 07065-0907 (US); MCCLEMENTS, William, L., Rahway, NJ 07065-0907 (US); JANSEN, Kathrin, U., Rahway, NJ 07065-0907 (US); SCHULTZ, Loren, D., Rahway, NJ 07065-0907 (US); CHEN, Ling, Rahway, NJ 07065-0907 (US); WANG, Xin-Min, Rahway, NJ 07065-0907 (US)
(74) Representative: Horgan, James Michael Frederic
(86) International application number: PCT/US2000/022932
(87) International publication number: WO 2001/014416

(56) References cited:
- WO-A-96/00583
- WO-A-96/39178
- US-A- 5 679 509
- ZHOU JIAN ET AL: "Papillomavirus capsid protein expression level depends on the match between codon usage and tRNA availability." JOURNAL OF VIROLOGY, vol. 73, no. 6, June 1999 (1999-06), pages 4972-4982, XP002164427 ISSN: 0022-538X
- AFGHAN R K ET AL: "Immune responses induced by vaccination with plasmid DNA containing the HPV16 E7 ORF." IMMUNOLOGY, vol. 95, no. SUPPL. 1, December 1998 (1998-12), page 106 XP000982724 6th Annual Congress of the British Society for Immunology;Harrogate, England, UK; December 1-4, 1998 ISSN: 0019-2805
- KOTECHA M T ET AL: "Humoral responses induced by vaccination with plasmid DNA containing the HPV16 L1 ORF." IMMUNOLOGY, vol. 95, no. SUPPL. 1, December 1998 (1998-12), page 107 XP000982725 6th Annual Congress of the British Society for Immunology;Harrogate, England, UK; December 1-4, 1998 ISSN: 0019-2805
- XU JIANQING ET AL: "Human papillomavirus 16 E7-specific CTL induction through mouse B7-1 costimulating with E7C subgene." ZHONGHUA WEISHENGWUXUE HE MIANYIXUE ZAZHI, vol. 19, no. 3, May 1999 (1999-05), pages 227-231, XP000989739 ISSN: 0254-5101
- SMAHEL MICHAL ET AL: "DNA vaccine against oncogenic hamster cells transformed by HPV16 E6/E7 oncogenes and the activated ras oncogene." ONCOLOGY REPORTS, vol. 6, no. 1, January 1999 (1999-01), pages 211-215, XP000989763 ISSN: 1021-335X
- NIMAKO MERCY ET AL: "Human papillomavirus-specific cytotoxic T lymphocytes in patients with cervical intraepithelial neoplasia grade III." CANCER RESEARCH, vol. 57, no. 21, 1 November 1997 (1997-11-01), pages 4855-4861, XP000982722 ISSN: 0008-5472
- BORYSIEWICZ L K ET AL: "A recombinant vaccinia virus encoding human papillomavirus types 16 and 18, E6 and E7 proteins as immunotherapy for cervical cancer." LANCET (NORTH AMERICAN EDITION), vol. 347, no. 9014, 1996, pages 1523-1527, XP000997454 ISSN: 0099-5355
- TOES RENE E M ET AL: "Protective anti-tumor immunity induced by vaccination with recombinant adenoviruses encoding multiple tumor-associated cytotoxic T lymphocyte epitopes in a string-of-beads fashion." PROCEEDINGS OF THE NATIONAL ACADEMY OF SCIENCES OF THE UNITED STATES, vol. 94, no. 26, 23 December 1997 (1997-12-23), pages 14660-14665, XP002164428 Dec. 23, 1997 ISSN: 0027-8424
- HE Z ET AL: "Viral recombinant vaccines to the E6 and E7 antigens of HPV-16." VIROLOGY, vol. 270, no. 1, 25 April 2000 (2000-04-25), pages 146-161, XP000989692 ISSN: 0042-6822
- CHEN CHIEN-HUNG ET AL: "Boosting with recombinant vaccinia increases HPV-16 E7-specific T cell precursor frequencies of HPV-16 E7-expressing DNA vaccines." VACCINE, vol. 18, no. 19, 3 April 2000 (2000-04-03), pages 2015-2022, XP004202497 ISSN: 0264-410X

## Description

### FIELD OF THE INVENTION

This invention relates to human papillomavirus (HPV) genes which have been codon-optimized for expression in a human cellular environment, and their use with adenoviral vectors and or plasmid vectors as vaccines.

### BACKGROUND OF THE INVENTION

Papillomavirus infections occur in a variety of animals, including humans, sheep, dogs, cats, rabbits, snakes, monkeys and cows. Papillomaviruses infect epithelial cells, generally inducing benign epithelial or fibroepithelial tumors at the site of infection. Papillomaviruses are species specific infective agents; a human papillomavirus cannot infect a non-human.

Papillomaviruses are small (50-60nm), nonenveloped, icosahedral DNA viruses what encode up to eight early and two late genes. The open reading frames (ORFs) of the virus are designated E1 to E7 and L1 and L2, where "E" denotes early and "L" denotes late. L1 and L2 code for virus capsid proteins. The early genes are associated with functions such as viral replication and cellular transformation.

In humans, different HPV types cause distinct diseases, ranging from benign warts (for examples HPV types 1, 2, 3) to highly invasive genital and anal carcinomas (HPV types 16 and 18). At present there is not a satisfactory therapeutic regimen for these diseases.

Immunological studies in animals have shown that the production of neutralizing antibodies to papillomavirus antigens prevents infection with the homologous virus. However, development of a vaccine has been hindered by the difficulties associated with culture of the papillomavirus *in vitro.*

Vaccination is an effective form of disease prevention and has proven successful against several types of viral infection. However, to date, attempts to generate an effective HPV vaccine have not been entirely successful.

### SUMMARY OF THE INVENTION

This invention relates to oligonucleotides which encode a human papillomavirus (HPV) protein which has been codon-optimized for efficient expression in a host cell; preferably the oligonucleotides are DNA. In one embodiment, the polynucleotides encode a protein which retains its wild-type amino acid sequence. In an alternate embodiment, the polynucleotides encode a mutated form of a HPV protein which has reduced protein function as compared to wild-type protein, but which maintains immunogenicity.

In preferred embodiments, the protein is selected from the group consisting of: L1, L2, E1, E2, E4, E5, E6 and E7 proteins. Particularly preferred are L1, L2, E2, and E7 proteins.

Another aspect of this invention is a vector carrying the polynucleotides encoding a codon-optimized HPV protein. Yet another aspect of this invention are host cells containing these vectors.

In a preferred embodiment, the vector is an adenoviral vector. In a particularly preferred embodiment, the adenoviral vector is a vaccine vector comprising an adenoviral genome with a deletion in the E1 region, and an insert in the E1 region, wherein the insert comprises an expression cassette comprising:
a) a polynucleotide encoding an HPV protein selected from the group consisting of L1, E1, E2, and E7 proteins, wherein the polynucleotide is codon-optimized for expression in a human host cell; and
b) a promoter operably linked to the polynucleotide.

Another type of vector which is envisioned by this invention is a shuttle plasmid vector comprising a plasmid portion and an adenoviral portion, the adenoviral portion comprising: an adenoviral genome with a deletion in the E1 region, and an insert in the E1 region, wherein the insert comprises an expression cassette comprising:
a) a polynucleotide encoding an HPV protein selected from the group consisting of L1, E1, E2, and E7 proteins, wherein the polynucleotide is codon-optimized for expression in a human host cell; and
b) a promoter operably linked to the polynucleotide.

This invention also is directed to plasmid vaccine vectors, which comprise a plasmid portion and an expressible cassette comprising
a) a polynucleotide encoding an HPV protein selected from the group consisting of L1, E1, E2, and E7 proteins, wherein the polynucleotide is codon-optimized for expression in a human host cell; and
b) a promoter operably linked to the polynucleotide.

This invention also relates to vaccine compositions comprising a vector which carries the oligonucleotides to a human host, and allows for expression of the encoded protein. The protein is expressed in an amount sufficient to induce an immune response. In preferred embodiments, the vector is a plasmid vector or an adenoviral vector.

This invention also relates to a method of making a HPV protein comprising expressing in a host cell a synthetic polynucleotide encoding a human papillomavirus (HPV) protein, or mutated form of a HPV protein which has reduced protein function as compared to wild-type protein, but which maintains immunogenicity, the polynucleotide sequence comprising codons optimized for expression in a human host.

### BRIEF DECRYPTION OF THE DRAWINGS

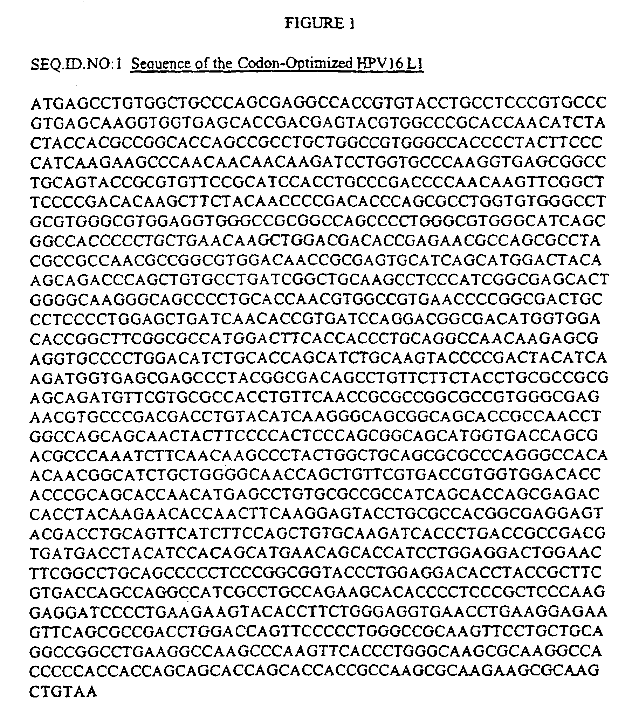

FIGURE 1 is the nucleotide sequence of a codon-optimized HPV16 L1 gene (SEQ.ID.NO:1).

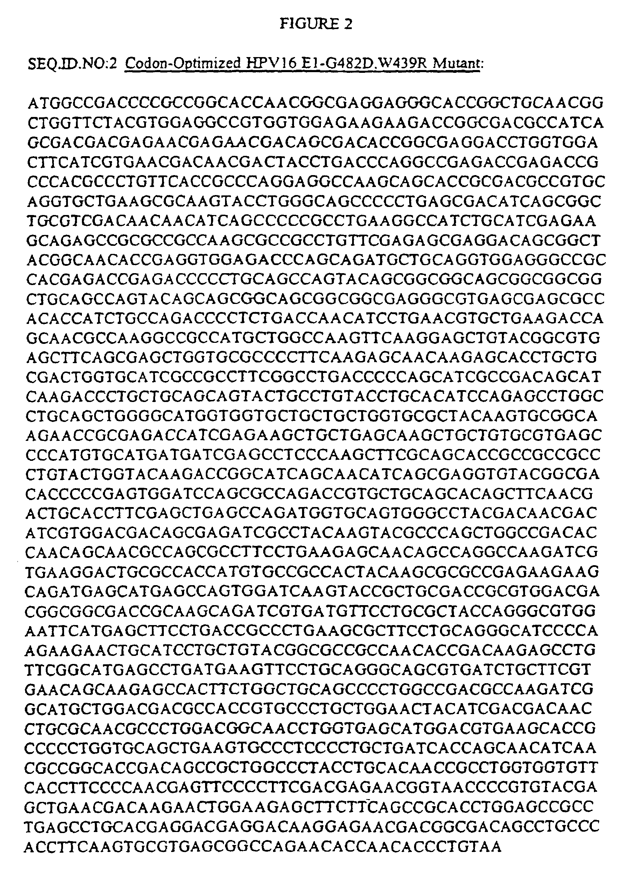

FIGURE 2 is the nucleotide sequence of a codon-optimized HPV 16 E1 gene (SEQ.ID.NO:2). In this particular sequence, there are further mutations which changes the amino acid sequence of the expressed protein-- the glycine residue at position 428 has been converted to aspartic acid, and the tryptophan residue at position 439 is now arginine.

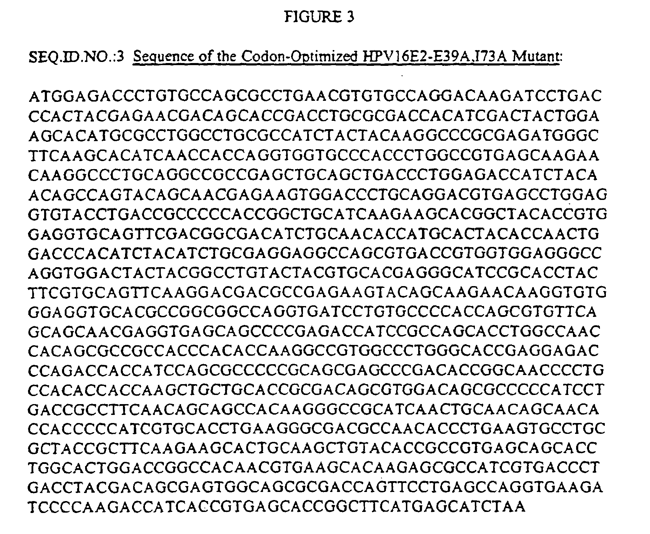

FIGURE 3 is the nucleotide sequence of a codon-optimized HPV 16 E2 gene (SEQ.ID.NO:3). In this particular sequence, the glutamic acid residue at position 39 has been changed to an alanine, and the isoleucine residue at position 73 has also been changed to an alanine.

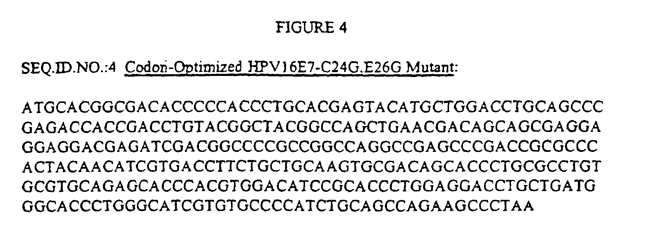

FIGURE 4 is the nucleotide sequence of a codon-optimized HPV 16 E7 gene.(SEQ.ID.NO:4). In this particular sequence, the cysteine residue at position 24 has been changed to glycine, and the glutamic acid residue at position 26 has been changed to a glycine.

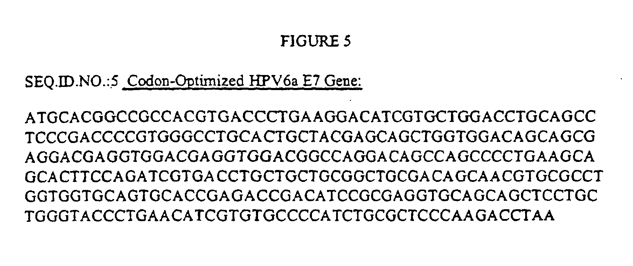

FIGURE 5 is the nucleotide sequence of a codon-optimized HPV6a E7 gene (SEQ.ID.NO:5).

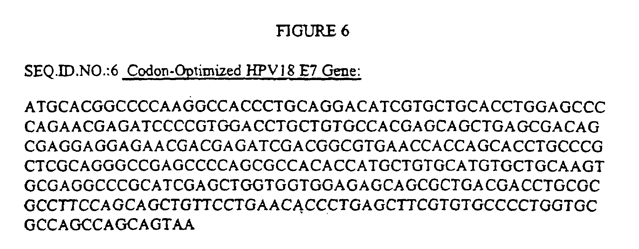

FIGURE 6 is the nucleotide sequence of a codon-optimized HPV18 E7 gene (SEQ.ID.NO:6).

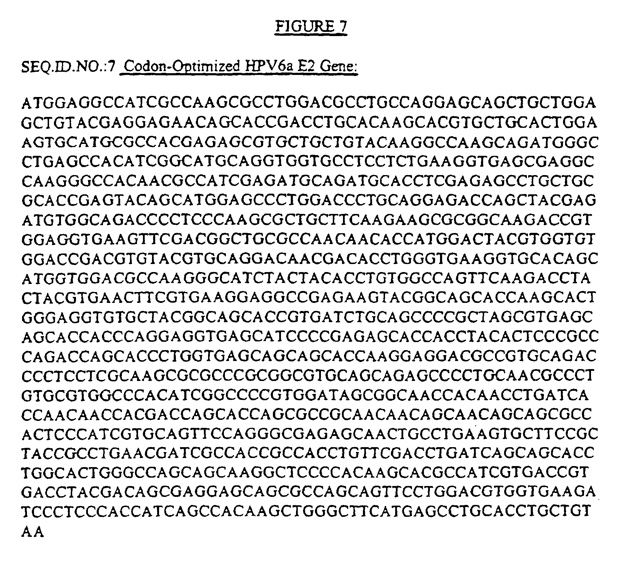

FIGURE 7 is the nucleotide sequence of a codon-optimized HPV6a E2 gene (SEQ.ID.NO:7).

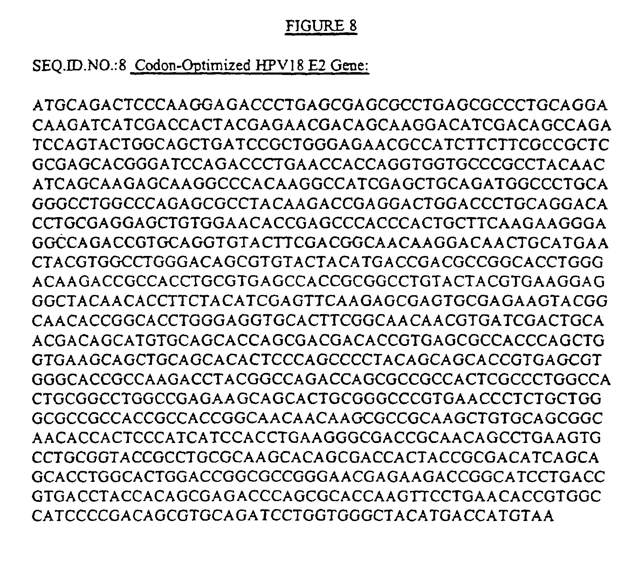

FIGURE 8 is the nucleotide sequence of a codon-optimized HPV18 E2 gene (SEQ.ID.NO:8).
FIGURE 9 shows the results of immunoblot analysis of lysates of 293 cells transiently-transfected with native (lanes d, e, f) or synthetic (a, b, c) HPV16 L1 sequences in the expression vector V1Jns.
FIGURE 10 shows the results of immunoblot analysis of lysates of 293 cells transiently-transfected with native or synthetic HPV16 E1 sequences in the expression vector V1Jns. Lanes a and d contain native HPV16 E1 sequences; lanes b and e contain synthetic HPV16 E1, and lane c is a mock-transfected control.
FIGURE 11 shows the results of immunoblot analysis of lysates of 293 cells transiently-transfected with native or synthetic HPV16 E2 sequences in the expression vector V1Jns. Lane a is mock-infected; lane b is lacZ control; lane c contains a synthetic HPV16 E2 isolate #6; lane d contains synthetic HPV16 E2 isolate #11, and lane e has native HPV16 E2.
FIGURE 12 shows the results of immunoblot analysis of lysates of 293 cells transiently-transfected with native or synthetic HPV16 E7 sequences in the expression vector V1Jns. Lane a is mock-infected; lane b is lacZ control; lane c contains synthetic HPV16 E7 isolate #2; lane d is synthetic HPV16 E7 isolate 4; and lane e is native HPV16 E7.
FIGURE 13 shows the results of immunoblot analysis of lysates of 293 cells transiently-transfected with synthetic HPV6a E7 sequences in the expression vector V1Jns. Lanes b and c contain synthetic HPV6a E7 sequences; lane d contains a lacZ control, and lane a is a mock-transfected control.
FIGURE 14 shows the results of immunoblot analysis of lysates of 293 cells transiently-transfected with synthetic HPV18 E7 sequences in the expression vector V1Jns. Lanes b, c, d, and e contain synthetic HPV18 E7 sequences; lane f contains synthetic HPV16 E7 as an antibody control, and lane a is a mock-transfected control.
FIGURE 15 shows the results of immunoblot analysis of lysates of 293 cells transiently-transfected with synthetic HPV6a E2 sequences in the expression vector V1Jns. Lanes a and b contain synthetic E2 sequences; lane c is a beta-gal control, and lane d is mock-transfected.
FIGURE 16 shows the results of immunoblot analysis of lysates of 293 cells transiently-transfected with synthetic HPV18 E2 sequences in the expression vector V1Jns. Lane a is beta-gal control; lane b is mock transfected; and lanes c and d have synthetic sequences.
FIGURE 17 is a table of oligonucleotides (SEQ.ID.NOS:9-32) used to generate synthetic HPV16 L1.
FIGURE 18 is a table of oligonucleotides (SEQ.ID.NOS:33-64) used to generate synthetic HPV16 E1.

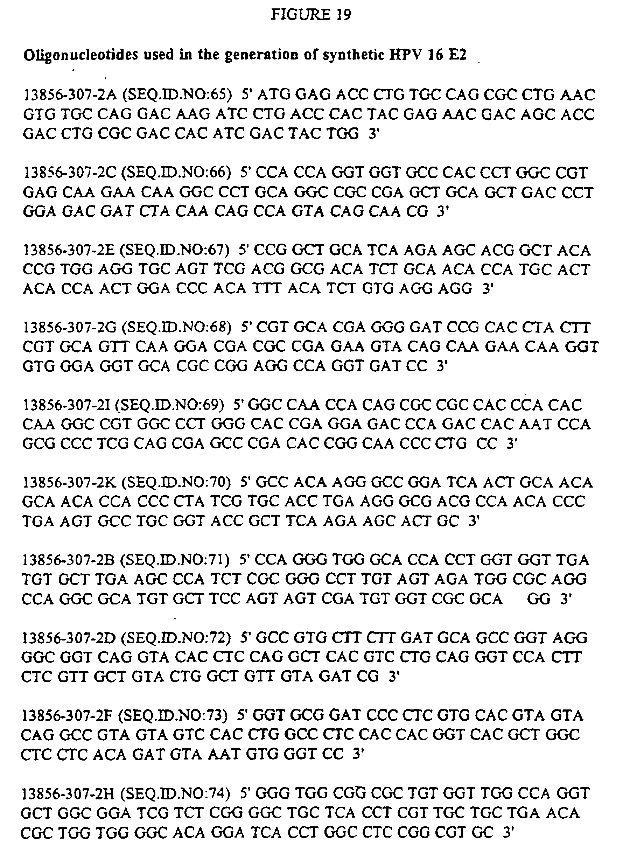

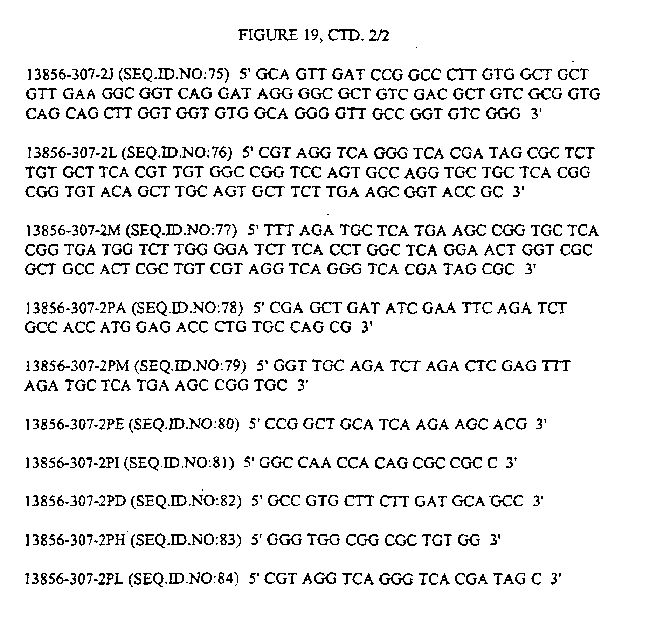

FIGURE 19 is a table of oligonucleotides (SEQ.ID.NOS:65-84) used to generate synthetic HPV16 E2.
FIGURE 20 is a table of oligonucleotides (SEQ.ID.NOS:85-90) used to generate synthetic HPV16 E7.
FIGURE 21 is a table of oligonucleotides (SEQ.ID.NOS:91-96) used to generate synthetic HPV6a E7.
FIGURE 22 is a table of oligonucleotides (SEQ.ID.NOS:97-102) used to generate synthetic HPV18 E7.
FIGURE 23 is a table of oligonucleotides (SEQ.ID.NOS:103-126) used to generate synthetic HPV6a E2.
FIGURE 24 is a table of oligonucleotides (SEQ.ID.NOS:127-150) used to generate synthetic HPV 18 E2.
FIGURE 25 is a Western blot of JCL-031 cell lysate. Cell lysate was prepared from JCL-031 cells grown in selection medium containing 400µg/mL G418. The immunoblot was developed with anti-HPV 16 E2 (goat 248) antisera. Positions of molecular weight markers are indicated.
FIGURE 26 shows protection from JCL-031 cell-induced tumor outgrowth. E2 DNA- or control DNA-immunized mice were challenged by subcutaneous injection of 5 X10⁵ JCL-031 cells into the left inguinal region. Beginning five days after this challenge, all animals were observed at two or three day intervals until four weeks after inoculation. Tumors were detected and monitored by visual inspection, palpation of the inguinal region, and measurement of tumor diameter with calipers.

The term "promoter" as used herein refers to a recognition site on a DNA strand to which the RNA polymerase binds. The promoter forms an initiation complex with RNA polymerase to initiate and drive transcriptional activity. The complex can be modified by activating sequences termed "enhancers" or inhibiting sequences termed "silencers".

The term "cassette" refers to the sequence of the present invention which contains the nucleic acid sequence which is to be expressed. The cassette is similar in concept to a cassette tape; each cassette has its own sequence. Thus by interchanging the cassette, the vector will express a different sequence. Because of the restrictions sites at the 5' and 3' ends, the cassette can be easily inserted, removed or replaced with another cassette.

The term "vector" refers to some means by which DNA fragments can be introduced into a host organism or host tissue. There are various types of vectors including plasmid, virus (including adenovirus), bacteriophages and cosmids.

The term "effective amount" means sufficient vaccine composition is introduced to produce the adequate levels of the polypeptide, so that an immune response results. One skilled in the art recognizes that this level may vary.

"Synthetic" means that the HPV gene has been modified so that it contains codons which are preferred for human expression. In many cases, the amino acids encoded by the gene remain the same. In some embodiments, the synthetic gene may encode a modified protein.

The term "native" means that the gene contains the DNA sequence as found in occurring in nature. It is a wild type sequence of viral origin.

### DETAILED DESCRIPTION OF THE INVENTION

Synthetic DNA molecules encoding various HPV proteins are provided. The codons of the synthetic molecules are designed so as to use the codons preferred by the projected host cell, which is preferred embodiments is a human cell. The synthetic molecules may be used as a polynucleotide vaccine which provides effective immunoprophylaxis against papillomavirus infection through neutralizing antibody and cell-mediated immunity. The synthetic molecules may be used as an immunogenic composition. This invention provides polynucleotides which, when directly introduced into a vertebrate *in vivo,* including mammals such as primates and humans, induce the expression of encoded proteins within the animal.

The gene encoding a L1, E1, E2 and/or E7 from any serotype HPV can be modified in accordance with this invention. It is preferred that the HPV chosen be one which is known to cause a pathological condition in humans. For this reason, it is preferred that the HPV gene be selected from the group consisting of: HPV6a, HPV6b, HPV11, HPV16, HPV18, HPV31, HPV33, HPV35, HPV39, HPV45, HPV51, HPV52, HPV56, HPV58, HPV68 or variants thereof. The vaccine formulation of this invention may contain a mixture of HPV type protein genes (for example, genes from HPV6,11, 16 and 18), and/or it may also contain a mixture of protein genes (i.e. L1, E1, E2, and/or E7).

### Codon optimization

The wild-type sequences for many HPV genes are known. In accordance with this invention, HPV gene segments were converted to sequences having identical translated sequences but with alternative codon usage as defined by Lathe, 1985 "Synthetic Oligonucleotide Probes Deduced from Amino Acid Sequence Data: Theoretical and Practical Considerations" *J. Molec*. *Biol*. 183:1-12, which is hereby incorporated by reference. The methodology may be summarized as follows:
1. Identify placement of codons for proper open reading frame.
2. Compare wild type codon for observed frequency of use by human genes.
3. If codon is not the most commonly employed, replace it with an optimal codon for high expression in human cells.
4. Repeat this procedure until the entire gene segment has been replaced.
5. Inspect new gene sequence for undesired sequences generated by these codon replacements (e.g., "ATTTA" sequences, inadvertent creation of intron splice recognition sites, unwanted restriction enzyme sites, etc.) and substitute codons that eliminate these sequences.
6. Assemble synthetic gene segments and test for improved expression.

In this context, Zhou et al., 1999, Journal of Virology, vol. 73(6) : 4972-4982 disclose synthetic versions of BPV-1 L1 and L2 genes, substituting codous in L1 and L2 genes, less commonly used in mammalian genes, with codous preferentially used in mammalian systems and express said genes in COS-1 cells.

In accordance with this invention, it has been found that the use of alternative codons encoding the same protein sequence may remove the constraints on expression of HPV proteins by human cells.

These methods were used to create the following synthetic gene segments for various papillomavirus genes creating a gene comprised entirely of codons optimized for high level expression. While the above procedure provides a summary of our methodology for designing codon-optimized genes for DNA vaccines, it is understood by one skilled in the art that similar vaccine efficacy or increased expression of genes may be achieved by minor variations in the procedure or by minor variations in the sequence.

In some embodiments of this invention, alterations have been made (particularly in the E-protein native protein sequences) to reduce or eliminate protein function while preserving immunogenicity. Mutations which decrease enzymatic function are known. Certain alterations were made for purposes of expanding safety margins and/or improving expression yield. These modifications are accomplished by a change in the codon selected to one that is more highly expressed in mammalian cells. In the case of HPV16 E1, for example two mutations were introduced: glycine at amino acid number 482 was changed to aspartic acid by conversion of GGC to GAC; and tryptophan was changed to arginine at position 439 by conversion of TGG to CGC.

For HPV16 E2, conversion of glutamic acid at position 39 to alanine and isoleucine at position 73 to alanine by conversion of both codons each to GCC.

For HPV16 E7, conversion of cysteine at position 24 to glycine and glutamic acid at position 26 to glycine was permitted by alteration of TGC and the GAG respectively both to GGC.

The codon-optimized genes are then assembled into an expression cassette which comprises sequences designed to provide for efficient expression of the protein in a human cell. The cassette preferably contains the codon-optimized gene, with related transcriptional and translations control sequences operatively linked to it, such as a promoter, and termination sequences. In a preferred embodiment, the promoter is the cytomegalovirus promoter with the intron A sequence (CMV-intA), although those skilled in the art will recognize that any of a number of other known promoters such as the strong immunoglobulin, or other eukaryotic gene promoters may be used. A preferred transcriptional terminator is the bovine growth hormone terminator, although other known transcriptional terminators may also be used. The combination of CMVintA-BGH terminator is particularly preferred.

Examples of preferred gene sequences are given in SEQ.ID.NOS: 1-8.

### VECTORS

In accordance with this invention, the expression cassette encoding at least one HPV protein is then inserted into a vector. The vector is preferably a plasmid or an adenoviral vector, although linear DNA linked to a promoter, or other vectors, such as adeno-associated virus or a modified vaccinia virus vector may also be used.

If the vector chosen is an adenovirus, it is preferred that the vector be a so-called first-generation adenoviral vector. These adenoviral vectors are characterized by having a non-functional E1 gene region, and preferably a deleted adenoviral E1 gene region. In some embodiments, the expression cassette is inserted in the position where the adenoviral E1 gene is normally located. In addition, these vectors optionally have a non-functional or deleted E3 region. The adenoviruses can be multiplied in known cell lines which express the viral E1 gene, such as 293 cells, or PerC.6 cells.

For convenience in manipulating the adenoviral vector, the adenovirus may be in a shuttle plasmid form. This invention is also directed to a shuttle plasmid vector which comprises a plasmid portion and an adenovirus portion, the adenovirus portion comprising an adenoviral genome which has a deleted E1 and optional E3 deletion, and has an inserted expression cassette comprising at least one codon-optimized HPV gene. In preferred embodiments, there is a restriction site flanking the adenoviral portion of the plasmid so that the adenoviral vector can easily be removed. The shuttle plasmid may be replicated in prokaryotic cells or eukaryotic cells.

Standard techniques of molecular biology for preparing and purifying DNA constructs enable the preparation of the adenoviruses, shuttle plasmids and DNA immunogens of this invention.

If the vector chosen is plasmid DNA, it is preferred that the vector contain one or more promoters recognized by mammalian or insect cells. In a preferred embodiment, the plasmid would contain a strong promoter such as, but not limited to the CMV promoter. The gene to be expressed would be linked to such a promoter. An example of such a plasmid would be the mammalian expression plasmid V1Jns as described (J. Shiver et. al. 1996, in *DNA Vaccines,* eds., M. Liu, et al. N.Y. Acad. Sci., N.Y., 772:198-208 and is herein incorporated by reference).

In some embodiment of this invention, the both the vaccine plasmid and the adenoviral vectors may be administered to a vertebrate in order to induce an immune response. In this case, the two vectors are administered in a "prime and boost" regimen. For example the first type of vector is administered, then after a predetermined amount of time, for example, 1 month, 2 months, six months, or other appropriate interval, a second type of vector is administered. Preferably the vectors carry expression cassettes encoding the same polynucleotide or combination of polynucleotides.

Thus, another aspect of this invention is a method for inducing an immune response against human papillomavirus in a vertebrate, comprising
A) introducing into the vertebrate a first vector comprising a polynucleotide encoding an HPV protein selected from the group consisting of L1, E1, E2, and E7 proteins, wherein the polynucleotide is codon-optimized for expression in a human host cell;
B) allowing a predetermined amount of time to pass; and
C) introducing into the vertebrate a second vector comprising adenoviral vaccine vector comprising an adenoviral genome with a deletion in the E1 region, and an insert in the E1 region, wherein the insert comprises an expression cassette comprises
   i) a polynucleotide encoding an HPV protein selected from the group consisting of L1, E1, E2, and E7 proteins, wherein the polynucleotide is codon-optimized for expression in a human host cell; and
   ii) a promoter operably linked to the polynucleotide.

In general, is preferred that the first vector be a plasmid vaccine vector and the second vector be an adenoviral vector. Thus this invention is directed to a method for inducing immune responses in a vertebrate comprising:
A) introducing into the vertebrate a plasmid vaccine, wherein the plasmid vaccine comprises a plasmid portion and an expression cassette portion, the expression cassette portion comprising:
   i) a polynucleotide encoding an HPV protein selected from the group consisting of L1, E1, E2, and E7 proteins, wherein the polynucleotide is codon-optimized for expression in a human host cell; and
   ii) a promoter operably linked to the polynucleotide;
B) allowing a predetermined amount of time to pass; and
C) introducing into the vertebrate an adenoviral vaccine vector comprising an adenoviral genome with a deletion in the E1 region, and an insert in the E1 region, wherein the insert comprises an expression cassette comprising:
   i) a polynucleotide encoding an HPV protein selected from the group consisting of L1, E1, E2, and E7 proteins or mutant forms thereof, wherein the polynucleotide is codon-optimized for expression in a human host cell; and
   ii) a promoter operably linked to the polynucleotide.

In yet another embodiment of the invention, the codon-optimized genes may be introduced into a recipient by way of a plasmid or adenoviral vector, as a "prime", and then a "boost" is accomplished by introducing into the recipient a polypeptide or protein which is essentially the same as that which is encoded by the codon-optimized gene. Fragments of a full length protein may be substituted, especially those which are immunogenic and/or include an epitope.

The amount of expressible DNA or transcribed RNA to be introduced into a vaccine recipient will depend partially on the strength of the transcriptional and translational promoters used and on the immunogenicity of the expressed gene product. In general, an immunologically or prophylactically effective dose of about 1 ng to 100 mg, and preferably about 10 µg to 300 µg of a plasmid vaccine vector is administered directly into muscle tissue. An effective dose for recombinant adenovirus is approximately 10⁶-10¹² particles and preferably about 10⁷-10¹¹ particles. Subcutaneous injection, intradermal introduction, impression through the skin, and other modes of administration such as intraperitoneal, intravenous, or inhalation delivery are also contemplated. It is also contemplated that booster vaccinations may be provided. Parenteral administration, such as intravenous, intramuscular, subcutaneous or other means of administration with adjuvants such as interleukin-12 protein, concurrently with or subsequent to parenteral introduction of the vaccine of this invention is also advantageous.

The vaccine vectors of this invention may be naked, that is, unassociated with any proteins, adjuvants or other agents which impact on the recipients' immune system. In this case, it is desirable for the vaccine vectors to be in a physiologically acceptable solution, such as, but not limited to, sterile saline or sterile buffered saline or the DNA may be associated with an adjuvant known in the art to boost immune responses, such as a protein or other carrier. Agents which assist in the cellular uptake of DNA, such as, but not limited to, calcium ions, may also be used to advantage. These agents are generally referred to herein as transfection facilitating reagents and pharmaceutically acceptable carriers. Techniques for coating microprojectiles coated with polynucleotide are known in the art and are also useful in connection with this invention.

The following examples are offered by way of illustration and are not intended to limit the invention in any manner.

### EXAMPLES

### EXAMPLE 1

### Synthetic Gene Construction

Synthetic gene sequences for human papillomavirus proteins L1, E1, E2, and E7 were generated by reverse translation of amino acid sequences using the most frequently used codons found in highly expressed mammalian genes. (R. Lathe, 1985, *J. Mol*. *Biol*. 183:1-12, which is hereby incorporated by reference). Some adjustments to these codon-optimized sequences were made to introduce or remove restriction sites. Oligonucleotides based on these sequences were chemically synthesized (Midland Certified Reagents; Midland, TX) and assembled by PCR amplification. (J. Haas et. al., 1996, *Current Biology* 6:315-324; and *PCR Protocols*, M. Innis, et al, eds., Academic Press, 1990, both of which are hereby incorporated by reference).

Full-length sequences were cloned into the mammalian expression vector V1Jns (J. Shiver et. al. 1996, in *DNA Vaccines,* eds., M. Liu, et al. N.Y. Acad. Sci., N.Y., 772:198-208, which is hereby incorporated by reference) and sequenced by standard methodology. In cases where the actual sequence differed from the expected and resulted in amino acid substitution, that sequence was corrected by PCR mutagenesis as previously described (*PCR Protocols,* M. Innis, et al, eds., Academic Press, 1990, pg 177-180).

Protein expression was evaluated by transient transfection of equal quantities of plasmid DNA into 293 (transformed embryonic human kidney) cells which were harvested at 48 hr post DNA addition. Cell lysates were normalized to provide equal protein loadings. Analysis was by indirect immunofluorescence or immunoblot (Western) analysis using sera prepared to each of the HPV proteins. (Current Protocols in Molecular Biology, eds., F. Ausabel, *et*. *Al*., John Wiley and Sons, 1998, which is hereby incorporated by reference).

### EXAMPLE 2

### Synthesis of HPV 16 L1

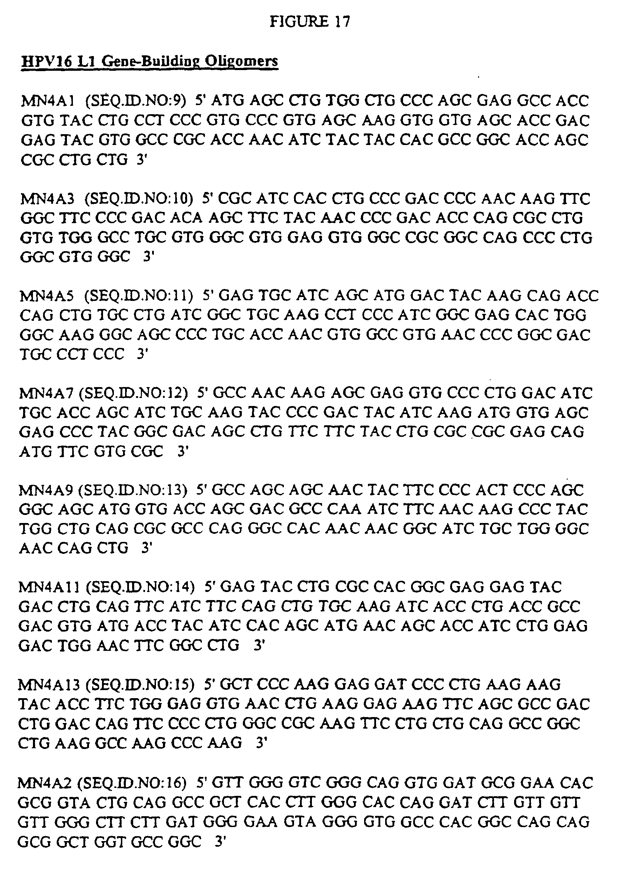

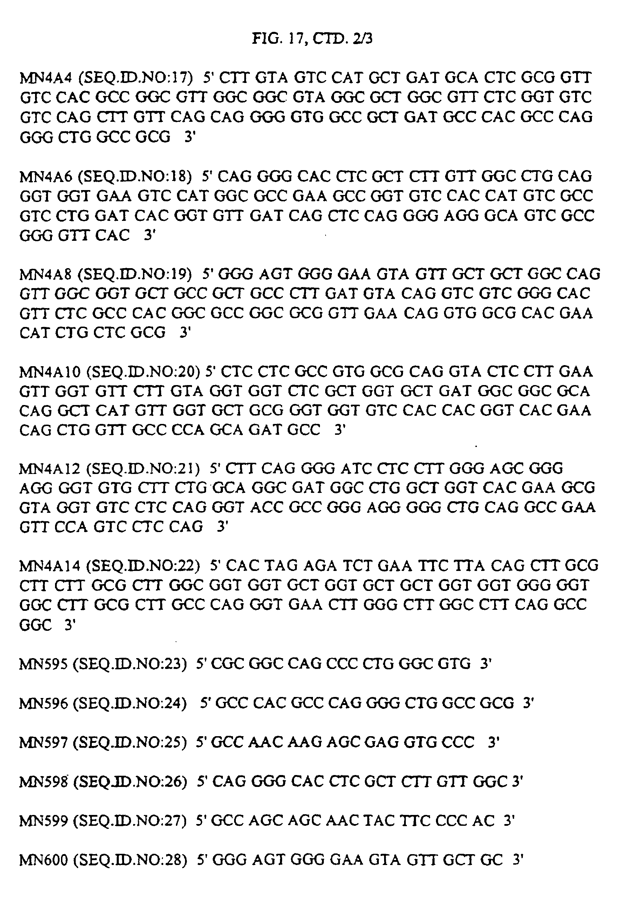

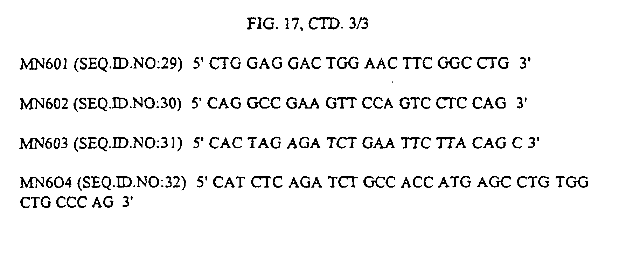

The gene encoding HPV 16 L1 was prepared by the annealing and extension of the 14 oligomers listed in FIGURE 17. Five separate extension reactions were performed to create fragments of the gene, designated L1A, L1B, L1C, L1D and L1E by PCR using conditions similar to those described in EXAMPLE 3 and 4, below.

L1A was constructed using oligomer sequences MN4A1 (SEQ.ID.NO:9), MN4A2 (SEQ.ID.NO:16) and MN4A3 (SEQ.ID.NO:10) which were amplified using the oligomers MN604 (SEQ.ID.NO:32) and MN596 (SEQ.ID.NO:24).

L1B was constructed using oligomer sequences MN4A4 (SEQ.ID.NO:17), MN4A5 (SEQ.ID.NO:11) and MN4A6 (SEQ.ID.NO:18) and were amplified using the oligomers MN595 (SEQ.ID.NO:23) and MN598 (SEQ.ID.NO:26).

L1C was created using oligomer sequences MN4A7 (SEQ.ID.NO:12) and MN4A8 (SEQ.ID.NO:19) and were amplified using the oligomers MN597 (SEQ.ID.NO:25) and MN602 (SEQ.ID.NO:30).

L1D was created using oligomer sequences MN4A9 (SEQ.ID.NO:13), MN4A10 (SEQ.ID.NO:20) and MN4A11 (SEQ.ID.NO:14) which were amplified using the oligomers MN597 (SEQ.ID.NO:25) and MN602 (SEQ.ID.NO:30).

L1E was created using oligomer sequences MN4A12 (SEQ.ID.NO:21), MN4A 13 (SEQ.ID.NO:15) and MN4A14 (SEQ.ID.NO:22) which were amplified using the oligomers MN601 (SEQ.ID.NO:29) and MN603 (SEQ.ID.NO:31).

Fragments L1A, L1B, L1C, L1D and L1E resulting from the PCR reactions were gel separated on low melting point agarose with the appropriately-sized products excised and purified using the Agarase™ method (Boehringer Mannheim Biochemicals) as recommended by the manufacturer. Fragments L1A, L1B and L1C were combined in a subsequent PCR reaction using oligomers MN604 (SEQ.ID.NO:32) and MN600 (SEQ.ID.NO:28) to assemble L1A-B-C; fragments L1D and L1E were assembled to L1D-E by subsequent PCR with the oligomers MN599 (SEQ.ID.NO:27) and MN603 (SEQ.ID.NO:31). The complete gene was then assembled by additional PCR reactions in which fragments L1A-B-C, L1D-E were combined with oligomers MN604 (SEQ.ID.NO:32) and MN603 (SEQ.ID.NO:31) in a final series of PCR reactions. The resulting 1.5 kb product was gel isolated, digested with Bgl II and subcloned into the V1Jns and sequenced. In instances where a mutation was observed, it was corrected by PCR mutagenesis as described in EXAMPLE 1. DNA was isolated from a clone with the correct HPV16 L1 DNA sequence and proper orientation within V1Jns for use in transient transfection assays as described in EXAMPLE 1.

### Transfection Results (HPV16 L1)

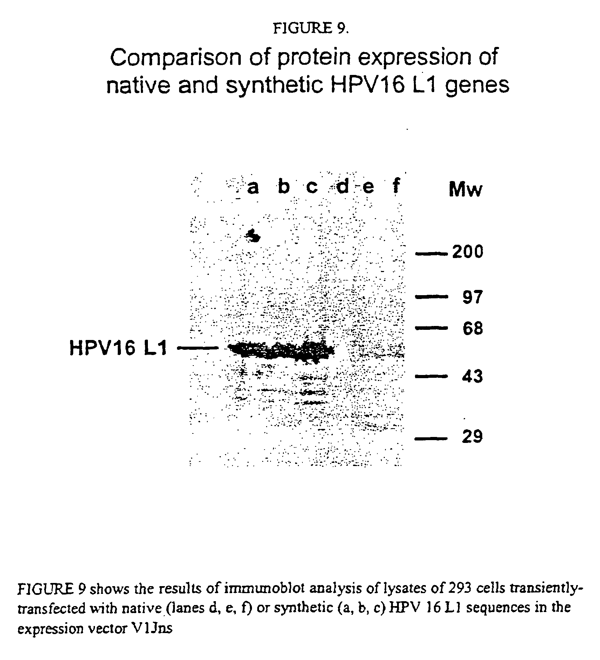

FIGURE 9 shows the HPV16 L1 immunoblot results of lysates of 293 cells transiently-transfected with the V1Jns plasmid containing either the native or the codon-optimized, synthetic HPV16 L1. Lanes a, b and c are the expression levels achieved using the synthetic HPV16 L1 expression construct. High levels of immunoreactive material are apparent in each of these lanes with the predominant band at approximately 55 kDa, consistent with the expected molecular weight for full-length HPV 16 L1. In contrast, virtually no immunoreactive material is apparent in the lanes containing lysates transfected with the native HPV16 L1/ V1Jns plasmid (lanes d, e, and f). Since all cell lysate loadings were normalized and equivalent DNA amounts were used in the transfections, these findings indicate that the synthetic gene sequence greatly increased the levels of HPV16 L1 protein accumulation relative to that of the native gene sequence.

### EXAMPLE 3

### Synthesis of HPV 16 E1

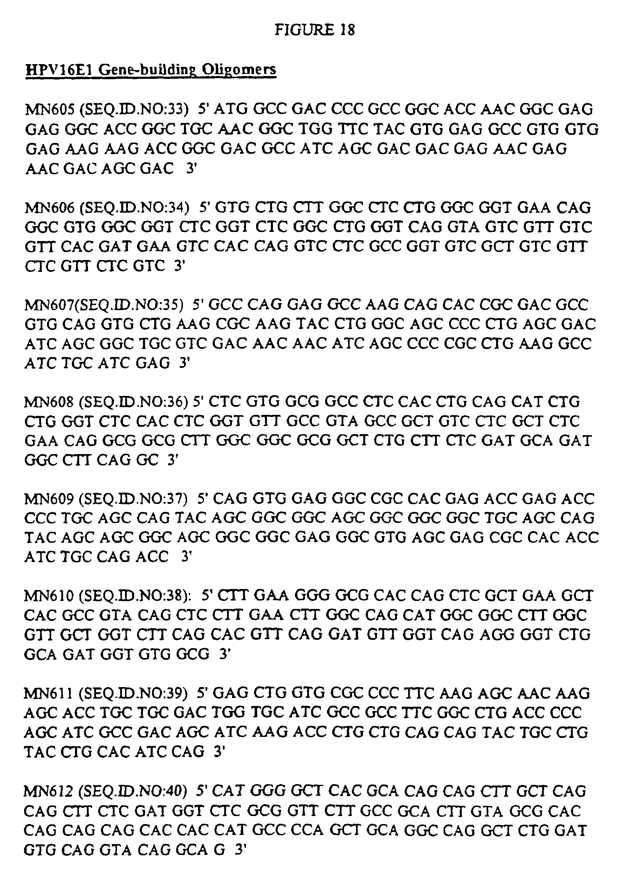

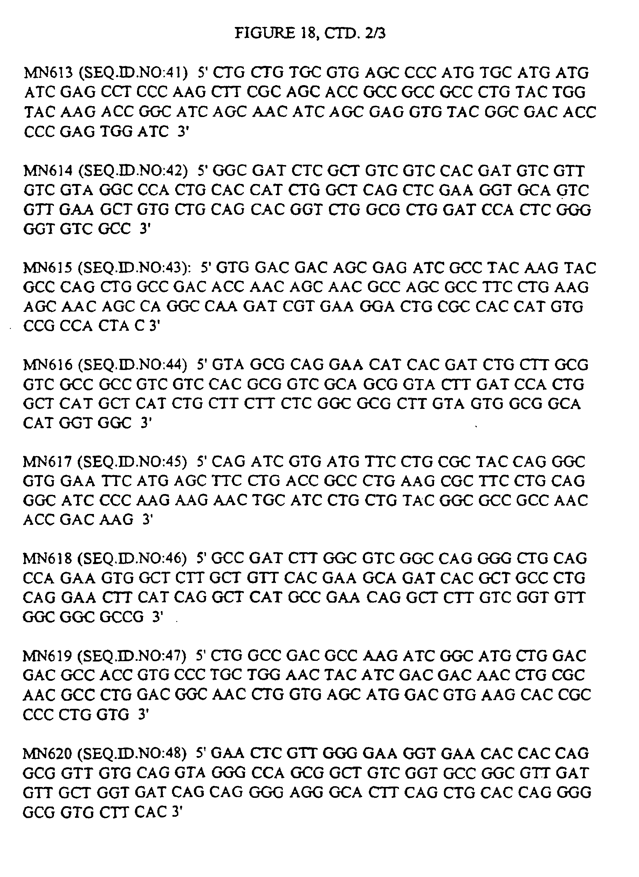

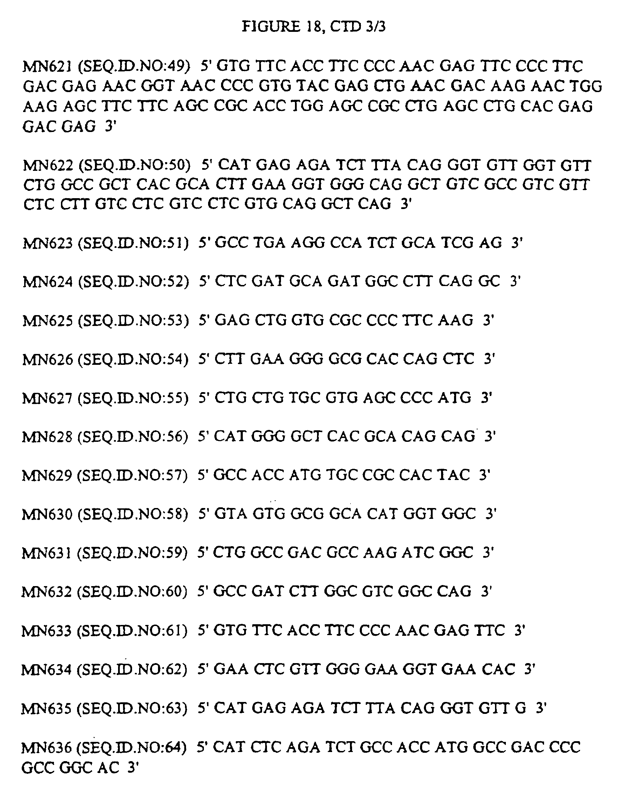

The gene encoding the modified form of HPV16 E1 was assembled from a series of fragments: E1A, E1B, E1C, E1D, E1E and E1F, using the oligomers listed in FIGURE 18. E1A was formed by assembly of oligomers MN605 (SEQ.ID.NO:33), MN606 (SEQ.ID.NO:34) and MN607 (SEQ.ID.NO:35) and amplified using oligomers MN636 (SEQ.ID.NO:64) and MN624 (SEQ.ID.NO:52).

E1B was formed by assembly of oligomers MN608 (SEQ.ID.NO:36), MN609 (SEQ.ID.NO:37) and MN610 (SEQ.ID.NO:38) which were amplified with oligomers MN623 (SEQ.ID.NO:51) and MN626 (SEQ.ID.NO:54).

E1C was formed by assembly of oligomers MN611 (SEQ.ID.NO:39) and MN612 (SEQ.ID.NO:40) which were amplified with oligomers MN625 (SEQ.ID.NO:53) and MN628 (SEQ.ID.NO:56).

E1D was formed by assembly of oligomers MN613 (SEQ.ID.NO:41), MN614 (SEQ.ID.NO:42) and MN615 (SEQ.ID.NO:43) which were amplified with oligomers MN627 (SEQ.ID.NO:55) and MN630 (SEQ.ID.NO:58).

E1E was formed by assembly of oligomers MN616 (SEQ.ED.NO:44), MN617 (SEQ.ID.NO:45) and MN618 (SEQ.ID.NO:46) which were amplified with oligomers MN629 (SEQ.ID.NO:57) and MN632 (SEQ.ID.NO:60).

E1F was formed by assembly of oligomers MN619 (SEQ.ID.NO:47), MN620 (SEQ.ID.NO:48) and MN621(SEQ.ID.NO:49) which were amplified with oligomers MN631 (SEQ.ID.NO:59) and MN635 (SEQ.ID.NO:63).

Products of these PCR reactions were gel isolated and combined in subsequent rounds of PCR to form a 2 kb gene fragment encoding HPV16 E1 using methods described above. The resulting HPV16 E1 was inserted into the V1Jns expression vector as above and utilized in transient transfection studies as described in EXAMPLE 1.

### Transfection Results (HPV16 E1)

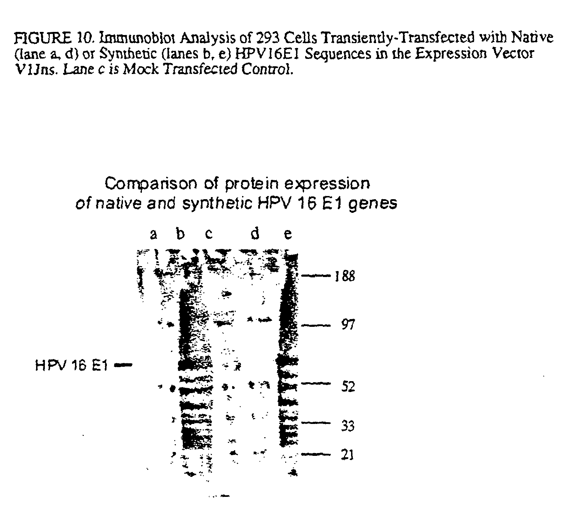

FIGURE 10 shows the HPV16 E1 immunoblot results of lysates of 293 cells transiently-transfected with the V1Jns plasmid containing either the native, or the codon-optimized, synthetic HPV16 E1. Lanes b and e are the expression levels achieved using the codon-optimized HPV16 E1 expression construct. High levels of HPV16 E1-specific immunostaining are apparent with a predominant band in lanes b and e at 72 kDa, consistent with the expected size for full-length HPV16 E1. In addition, there a number of smaller immunoreactive products which appear to be E1-specific as they are not observed in the mock transfected control (lane c).

A very different expression profile is observed in lysates of cells transfected with the native HPV16 E1/V1Jns construct, however. As shown in lanes a and d, only minimal amounts of immunoreactive material can be visualized which is not present in the mock transfection control. Since all cell lysate loadings were normalized and equivalent DNA amounts were used in the transfections these findings indicate that the synthetic gene sequence greatly increased the levels of HPV16 E1 protein accumulation relative to that of the native gene sequence.

### EXAMPLE 4

### Synthesis of HPV 16 E2

Fragment AD. A 50 µl reaction containing oligonucleotides 13856-307-2A (SEQ.ID.NO:65), 13856-307-2B (SEQ.ID.NO:71), 13856-307-2C (SEQ.ID.NO:66), and 13856-307-2D (SEQ.ID.NO:72), at 150 nM each, dNTPs 0.5 mM each, Native buffer (Stratagene; La Jolla, CA) and 1 µL Native Pfu DNA polymerase (Stratagene) was incubated in a GeneAmp 9700 thermocycler (Perkin Elmer Applied Biosystems; Foster City, CA) under the following conditions: 95°C, 2 min.; 20 cycles of 95°C, 45 sec.; 55°C, 45 sec.; and 72°C, 2.5 min. Added to the reaction were primers 13856-307-2PA (SEQ.ID.NO:78) and 13856-307-2PD (SEQ.ID.NO:82) to a final concentration of 400 nM each, and 1 µL of Native Pfu DNA polymerase. The mixture was incubated for 2 min at 95°C and then 25 cycles of 95°C, 45 sec; 55°C, 45 sec; and 72°C, 2.5 min. The gel-isolated full-length fragment AD was amplified for 20 cycles under the same conditions using primers 13856-307-2PA (SEQ.ID.NO:78) and 13856-307-2PD (SEQ.ID.NO:82).

Fragment EH. A 50 µl reaction containing oligonucleotides 13856-307-2E (SEQ.ID.NO:67), 13856-307-2F (SEQ.ID.NO:73), 13856-307-2G (SEQ.ID.NO:68), and 13856-307-2H (SEQ.ID.NO:74) at 150 nM each, dNTPs 0.5 mM each, Native buffer and 1 µL Native Pfu DNA polymerase was incubated under the following conditions: 95°C, 2 min; 20 cycles of 95°C, 2 min.; 45 sec.; 55°C, 45 sec.; and 72°C, 2.5 min. Added to the reaction were primers 13856-307-2PE (SEQ.ID.NO:80) and 13856-307-2PH (SEQ.ID. NO:83) to a final concentration of 400 nM each, and 1 µL of Native Pfu DNA polymerase. The mixture was incubated for 95°C, 2 min.; then 25 cycles of 95°C, 45 sec.; 55°C, 45 sec.; and 72°C, 2.5 min.

Fragment IL. A 50 µl reaction containing oligonucleotides 13856-307-2I (SEQ.ID.NO:69), 13856-307-2J (SEQ.ID.NO:75), 13856-307-2K (SEQ.ID.NO:70), and 13856-307-2L (SEQ.ID.NO:76) at 150 nM each, dNTPs 0.5 mM each, Native buffer and 1 µL Native Pfu DNA polymerase was incubated under the following conditions: 95°C, 2 min; 20 cycles of 95°C, 2 min.; 45 sec.; 55°C, 45 sec.; and 72°C, 2.5 min. Added to the reaction were primers 13856-307-2PI (SEQ.ID.NO:81) and 13856-307-2PL (SEQ.ID.NO. 84) to a final concentration of 400 nM each, and 1 µL of Native Pfu DNA polymerase. The mixture was incubated at 95°C, 2 min.; then 25 cycles of 95°C, 45 sec.; 55°C, 45 sec.; and 72°C, 2.5 min.

Fragment AH. A 50 µl reaction containing 1.5µl each of AD and EH PCR products, dNTPs 0.5 mM each, Native buffer and 1 µL Native Pfu DNA polymerase was incubated under the following conditions: 95°C, 2 min; 20 cycles of 95°C, 45 sec.; 55°C, 45 sec.; and 72°C, 3.5 min. Added to the reaction were primers 13856-307-2PA (SEQ.ID.NO:78) and 13856-307-2PH (SEQ.ID.NO:83) to a final concentration of 400 nM each, and 1 µL of Native Pfu DNA polymerase. The mixture was incubated at 95°C, 2 min.; then 25 cycles of 95°C, 45 sec.; 55°C, 45 sec.; and 72°C, 3.5 min.

Fragment IM. A 50 µl reaction containing 1µl of IL PCR product, oligonucleotides 13856-307-2M (SEQ.ID.NO:77) and 13856-307-2PI (SEQ.ID.NO:81) each at a final concentration of 400 nM, dNTPs 0.5 mM each, Native buffer and 1 µL Native Pfu DNA polymerase was incubated under the following conditions: 95°C, 2 min; 25 cycles of 95°C, 45 see.; 55°C, 45 sec.; and 72° C, 4 min.

Assembly of AM, full-length HPV16 E2. A 50 µl reaction containing 1.5µl each of fragments AH and IM, dNTPs 0.5 mM each, Native buffer and 1 µL Native Pfu DNA polymerase was incubated under the following conditions: 95°C, 2 min; 20 cycles of 95°C, 45 sec.; 55°C, 45 sec.; and 72°C, 4 min. Added to the reaction were primers 13856-307-2PA (SEQ.ID.NO:78) and 13856-307-2PM (SEQ.ID.NO:79) at a final concentration of 400 µM each, and 1 µL of Native Pfu DNA polymerase. The mixture was incubated at 95°C, 2 min.; then 25 cycles of 95° C, 45 sec.; 55°C, 45 sec.; and 72°C, 4 min. The resultant full-length fragment was isolated by electrophoresis through a 1.2% agarose gel the DNA recovered with a QIAquick column (Qiagen; Santa Clarita, CA) and subcloned into the expression vector V1Jns for evaluation.

### Transfection Results (HPV16 E2)

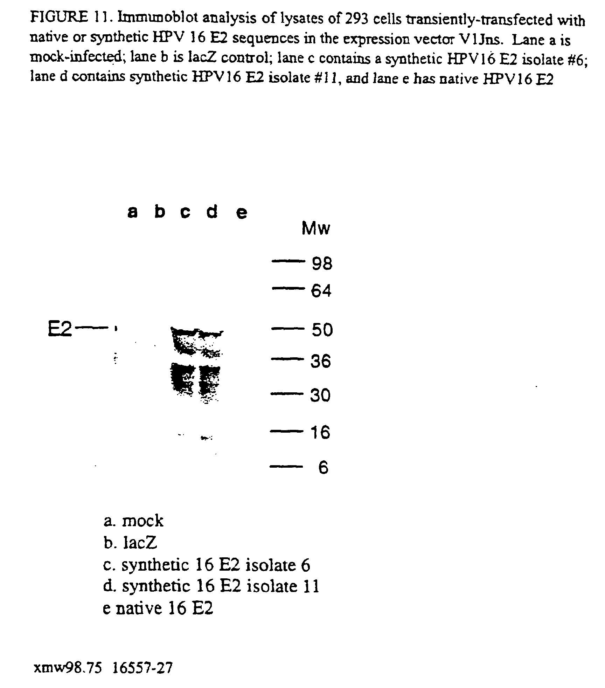

FIGURE 11 shows the HPV16 E2 immunoblot results of lysates of 293 cells transiently-transfected with the V1Jns plasmid containing either the native, or the synthetic HPV16 E2. Lanes c and d are the expression levels achieved using the codon-optimized HPV16 E2 expression construct. High levels of HPV16 E2-specific immunostaining are visible which appear to be E2-specific as they are not observed in the mock transfected control (lane c).

A very different expression profile is observed in lysates of cells transfected with the native HPV16 E2/V1Jns construct, however. As shown in lane e, no immunoreactive material can be visualized. Since all cell lysate loadings were normalized and equivalent DNA amounts were used in the transfections, these findings indicate that the synthetic gene sequence greatly increased the levels of HPV16 E2 protein accumulation relative to those of the native gene sequence.

### EXAMPLE 5

### Synthesis of HPV 16 E7

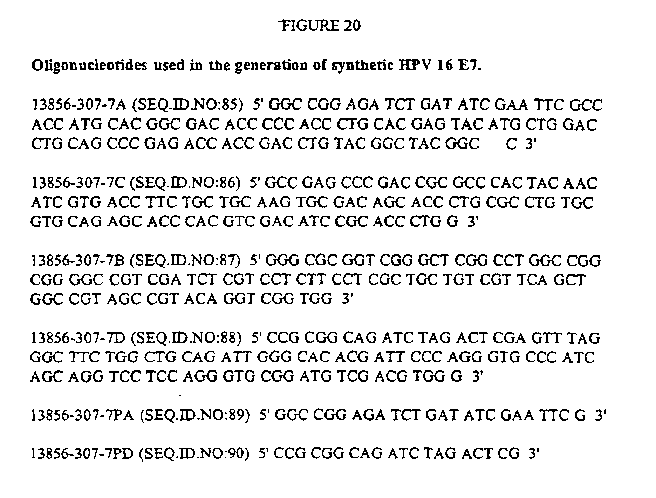

The gene encoding HPV16 E7 was assembled from a series of fragments, made using oligomers listed in FIGURE 20.

A 50 µl reaction containing oligonucleotides 13856-307-7A (SEQ.ID.NO:85), 13856-307-7B (SEQ.ID.NO:87), 13856-307-7C (SEQ.ID.NO:86), and 13856-307-7D (SEQ.ID.NO:88) at 150 nM each, dNTPs 0.5 mM each, Native buffer (Stratagene; La Jolla, CA) and 1 µL Native Pfu DNA polymerase (Stratagene) was incubated in a GeneAmp 9700 thermocycler (Perkin Elmer Applied Biosystems; Foster City, CA) under the following conditions: 95°C, 2 min; 20 cycles of 95°C, 45 sec.; 55°C, 45 sec. and 72°C, 2.5 min. Added to the reaction were primers 13856-307-7PA (SEQ.ID.NO:89) and 13856-307-7PD (SEQ.ID.NO:90) to a final concentration of 400 nM), and 1 µL of Native Pfu DNA polymerase. The mixture was incubated for 25 cycles of 95°C, 45 sec.; 55°C, 45 sec. and 72°C, 2.5 min.

The resultant full-length fragment was isolated by electrophoresis through a 1.2% agarose gel in TBE (Current Protocols in Molecular Biology, eds., F. Ausabel, *et. al*., John Wiley and Sons, 1998, which is hereby incorporated by reference), stained with ethidium bromide, cut from the gel and recovered through a GenElute column (Supleco; Bellefonte, PA) and resuspended in 20 µl water. The sequence was further amplified in a 51 µl reaction containing 2 µl of fragment, 0.5µM each of oligonucleotides 13856-307-7PA (SEQ.ID.NO:89) and 13856-307-7PD, (SEQ.ID.NO:90) dNTPs 0.5mM each, Native buffer and Native Pfu DNA polymerase. The reaction was subjected to 20 cycles of 95°C, 45 sec.; 55°C, 45 sec. and 72°C, 2.5 min. The final amplified product isolated by electrophoresis as described above; the DNA recovered with a QIAquick column (Qiagen; Santa Clarita, CA) and subcloned into V1Jns.

### Transfection Results (HPV16 E7)

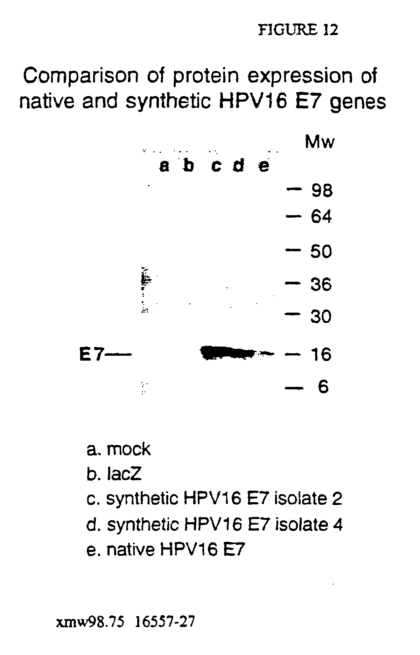

FIGURE 12 shows the HPV16 E7 immunoblot results of lysates of 293 cells transiently-transfected with the V1Jns plasmid containing either the native (lane e) or synthetic HPV16 E7 (lanes c and d). High levels of HPV16 E7-specific immunostaining are visible in the synthetic HPV16 E7 gene cell lysate lanes which are considerably more intense in appearance than that of the native HPV16 E7 gene cell lysate (lane e). Lanes a and b are negative transfection controls which show the antibody staining is specific to HPV16 E7 sequences. Since all cell lysate loadings were normalized and equivalent DNA amounts were used in the transfections, these findings indicate that the synthetic gene sequence greatly increased the levels of HPV16 E2 protein accumulation relative to those of the native gene sequence.

### EXAMPLE 6

### Synthesis of the E7 and E2 -encoding genes from HPV6a and HPV18:

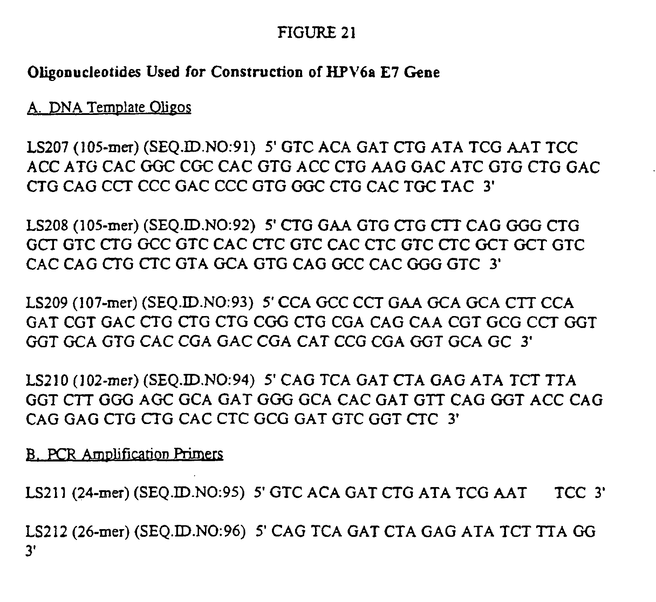

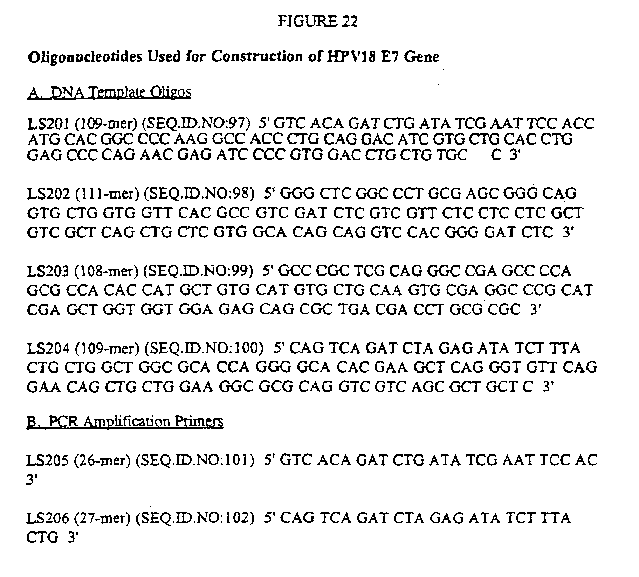

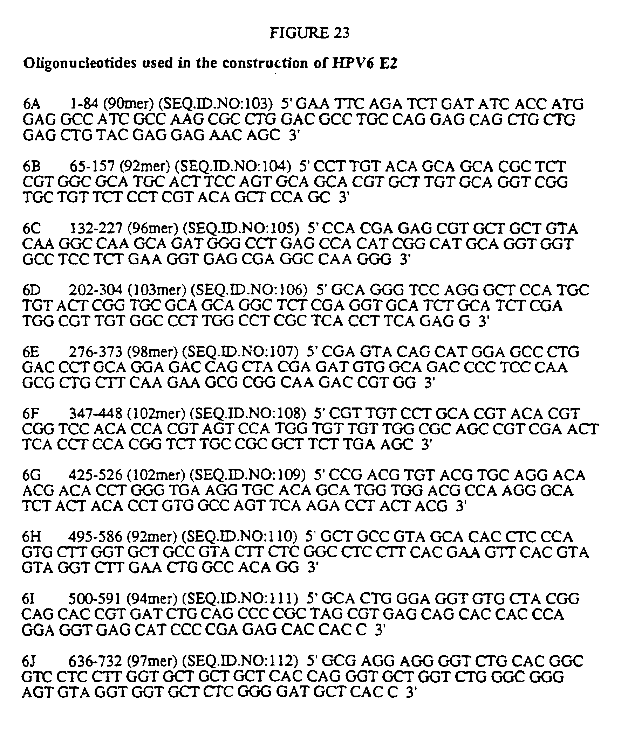

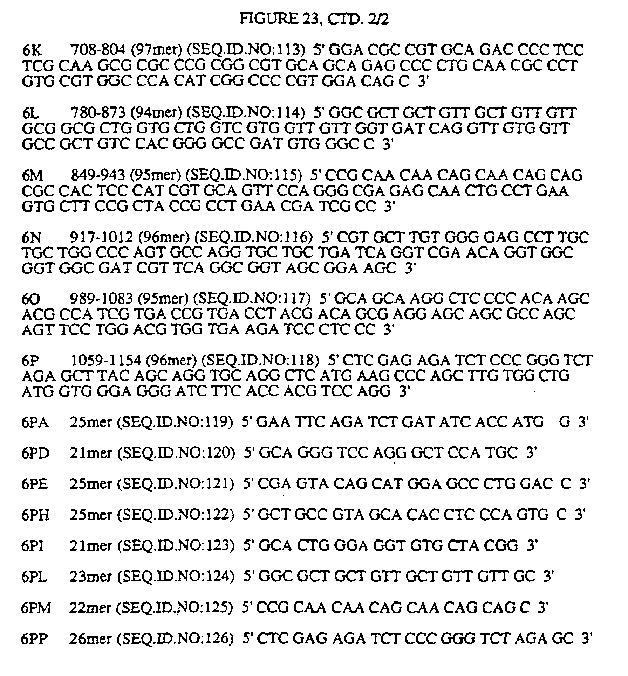

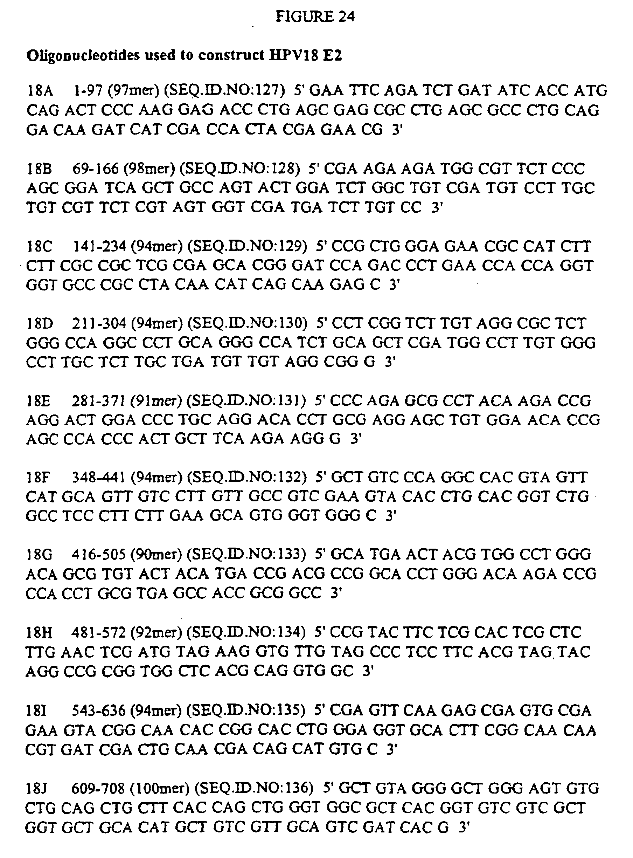

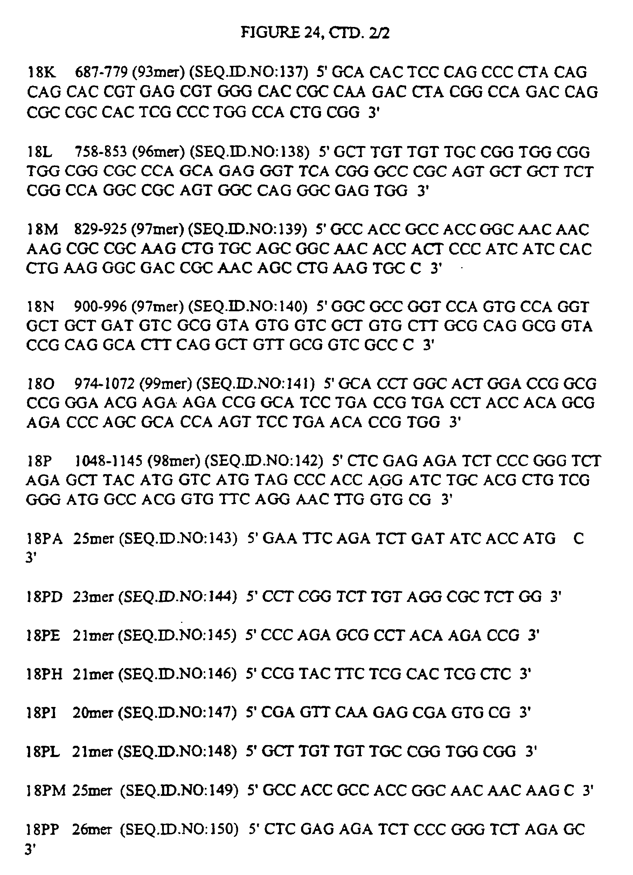

The genes encoding HPV6a E7 and HPV 18 E7 were constructed using similar methods as described in EXAMPLE 4, except that the oligomers used to create the HPV6a E7 and HPV 18 E7 genes contain the sequences listed in FIGURE 21 and FIGURE 22, respectively. The construction of the synthetic genes encoding HPV6a E2 and HPV18 E2 was performed in a similar manner as detailed in EXAMPLE 5 using the oligomer sequences listed in FIGURE 23 and FIGURE 24 respectively.

### Transfection Results: HPV6a E7 and HPV 18 E7

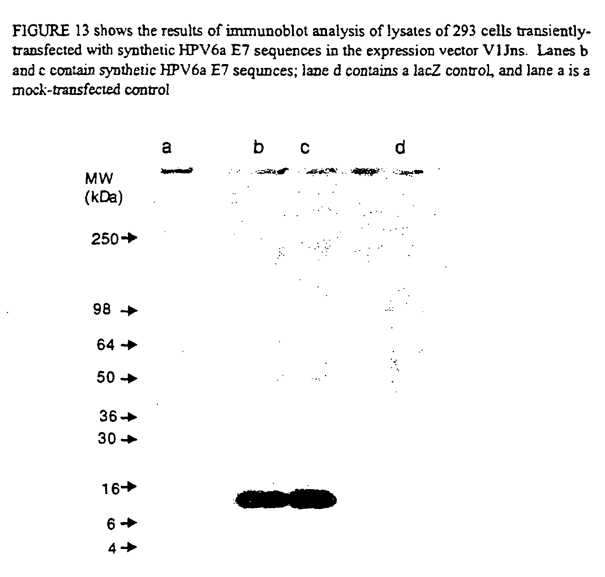

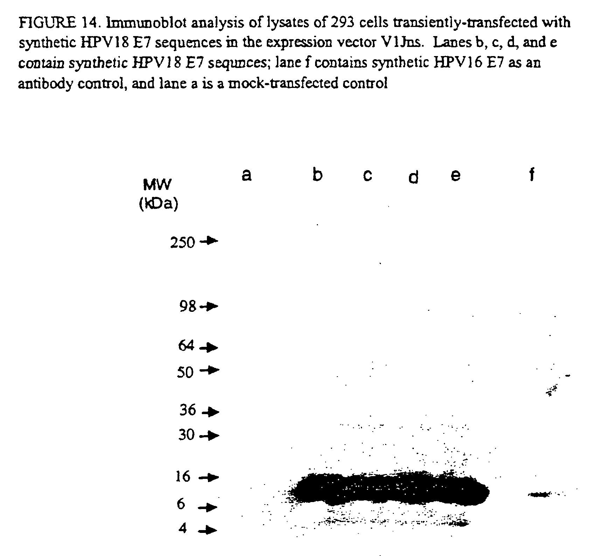

FIGURE 13 shows the HPV6a E7 immunoblot results of lysates of 293 cells transiently-transfected with the V1Jns plasmid containing synthetic HPV6a E7 (lanes b and c). High levels of HPV6a E7-specific immunostaining are visible in the region expected for full-length HPV6a E7. A similar profile is found in FIGURE 14 by HPV 18 E7 immunoblot analysis of lysates of 293 cells transiently-transfected with the V1Jns plasmid containing synthetic HPV6a E7 (lanes b, c, d and e). High levels of HPV 18 E7-specific immunostaining are visible where full-length HPV18 E7 would be found as indicated by the location of the purified HPV18 protein control (lane f). There does not appear to be any stained material in the negative control lane a which indicates the staining in the other lanes is HPV 18 E7-specific.

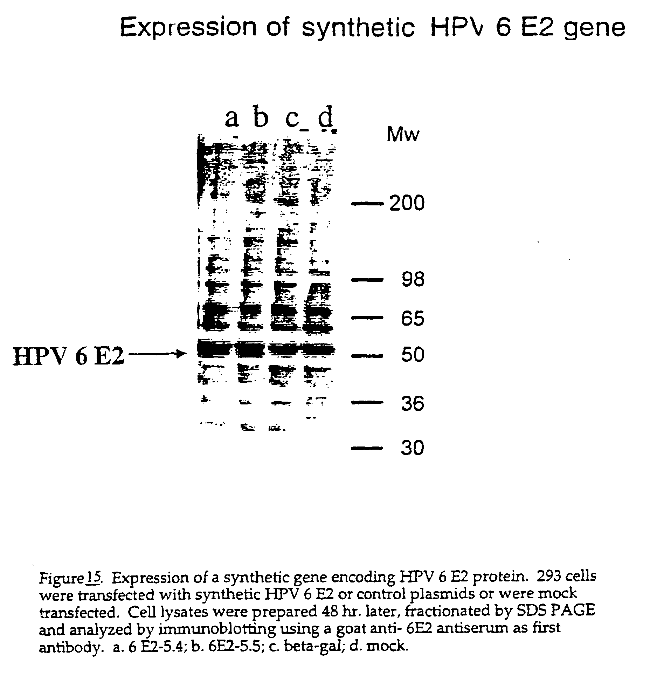

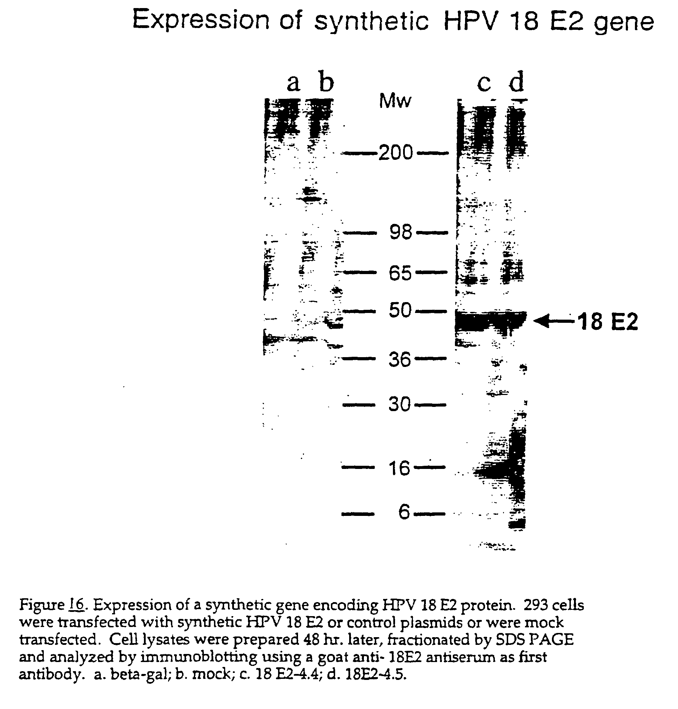

Expression of the synthetic gene encoding HPV6a E2 in V1Jns was evaluated by immunoblot analysis of transfected 293 cells which is shown in FIGURE 15. Lanes a and b are cell lysates of the synthetic HPV6a E2 transfectants; lanes c and d are negative controls. The analogous experiment is shown for HPV18 E2 expression in FIGURE 16. Lanes c and d are the cell lysates of transfections receiving the synthetic HPV18 E2 gene; lanes a and b are the negative controls. Both of these figures show measurable levels of E2 product accumulation when the codon-optimized, synthetic gene is expressed in mammalian cells.

These results indicate that the synthetic gene rebuilding is not limited to HPV16 genes. Rather, codon optimization of other HPV types also permits significant levels of E7 and E2 product accumulation in mammalian cells.

### EXAMPLE 7

### Construction of replication-defective FG-Ad expressing HPV antigen

### Starting vectors

Shuttle vector pHCMVIBGHpA1 contains Ad5 sequences from bp1 to bp 341 and bp 3534 to bp 5798 with a expression cassette containing human cytomegalovirus (HCMV) promoter plus intron A and bovine growth hormone polyadenylation signal.

The adenoviral backbone vector pAdE1-E3- (also named as pHVad1) contains all Ad5 sequences except those nucleotides encompassing the E1 and E3 region.

### Construction of Ad5. HPV16 E2

1. Construction of adenoviral shuttle plasmid pA1-CMVI-HPV16 E2 containing HPV16 E2 under the control of human CMV promoter and intron A.
   The HPV16 E2 insert was excised from pV1JNS-HPV16 E2 by restriction enzyme Bgl II, EcoRI and then cloned into Bgl II, EcoRI digested shuttle vector pHCMVIBGHpA1.
2. Homologous recombination to generate plasmid form of recombinant adenoviral vector pAd-CMVI-HPV16 E2 containing HPV16 E2 expression cassette.
   Shuttle plasmid pA1-CMVI- HPV16 E2 was digested with restriction enzymes BstZ17 and SgrAl and then co-transformed into *E. coli* strain BJ5183 with linearized (ClaI digested) adenoviral backbone plasmid pAdE1-E3-. A colony was verified by PCR analysis. The vector was transformed to competent *E. coli* HB101 for large quantity production of the plasmid.
3. Generation of recombinant adenovirus Ad.CMVI- HPV16 E2 in 293 cells.
   The pAd plasmid was linearized by restriction enzyme PacI and transfected to 293 cells using CaPO₄ method (Invitrogen kit). Ten days later, 10 plaques were picked and grown in 293 cells in 35-mm plates. PCR analysis of the adenoviral DNA showed virus were positive for HPV16 E2.
4. Evaluation of large scale recombinant adenovirus Ad.CMVI- HPV16 E2
   A selected clone was grown into large quantities through multiple rounds of amplification in 293 cells. Expression of HPV16 E2 was also verified by ELISA and Western blot analysis of the 293 or COS cells infected with the recombinant adenovirus. The recombinant adenovirus was used for evaluation in mice and rhesus monkeys.

### Method of Treatment

A person in need of therapeutic or prophylactic immunization against infection with human papillomavirus virus is injected with HPV DNA encoding all or part of; HPV L1, E1, E2, E4 or E7 and combinations thereof. The injection may be i.p., subcutaneous, intramuscular or intradermal. The HPV DNA may be used as a primer of the immune response or may be used as a booster of the immune response. The injection of DNA may antedate, coincide or follow injection of the person with a pharmaceutical composition comprising HPV virus like particles (containing only L1 protein or containing both L1 and L2 proteins, or containing mutant forms of one or more proteins), capsomeres, inactivated HPV, attenuated HPV, compositions comprising HPV-derived proteins, or combinations thereof.

### EXAMPLE 8

### The use of a synthetically-expressed HPV E protein as a model tumor antigen.

### Generation of a tumor cell line that expresses HPV 16 E2.

A Not I-Hind III restriction digest fragment containing the synthetic coding sequence for HPV 16 E2 (see above) was ligated with Not I, Hind III digested expression vector pBJ/neo/CCR2B which has a neomycin resistance marker and drives the expression of the transgene with the HCMV immediate early promoter. The resultant plasmid, pBJ-16 E2, was characterized by restriction digestion, sequence analysis of the cloning junctions, and the ability to induce E2 protein expression in transiently-transfected A293 or CT26 cells. A stable cell line was generated transfection of CT26 cells using Lipofectamine (Gibco BRL). CT26 cells, a fully-transformed line derived from a BALB/c mouse colon carcinoma, have been widely used to present model tumor antigens. (Brattain et al., 1980 *Cancer Research* 40:2142-2146; Fearon, E. et al.,1988 *Cancer Research,* 48:2975-2980; both of which are incorporated by reference).

After 48 hours, cells were trypsinized, diluted 1:10, 1:100, 1:1000 or 1:10000 into medium and plated in 100mm² plates. After 24 hours, the medium was replaced with selection medium containing 400µg/mL G418. After two to three weeks, well-isolated colonies of cells were recovered using cloning rings and transferred to 48-well plates. One clone was positive for E2 expression by immunoblot analysis and was subjected to two further rounds of cloning by limiting dilution. One G418 resistant, E2-positive clonal isolate was used to established the cell line JCL-031. (FIGURE 25).

When inoculated into (syngeneic) BALB/c mice by subcutaneous injection, JCL-031 cell induced tumors with the kinetics similar to those as the parental CT26 line. Cells cultured from recovered tumors were G418 resistant and expressed E2.

### Induction of immunity in mice by immunization with V1Jns:E2 DNA.

BALB/c mice were immunized multiple times by intramuscular injection with the DNA V1Jns:16E2 . Spleens from two randomly-chosen mice in each dose group were pooled, splenocytes prepared, and assayed in an murine interferon gamma Elispot assay. (Lalvani et al. 1997 *J*. *Exp*. *Med*. 186: 859-865; Forsthuber, T., et at 1996 *Science* 271: 1728-1730;. Chu, R. et al. 1997. *J. Exp. Med.* 186: 1623-1631, each of which is incorporated by reference.) Splenocyte cultures were incubated at 37°C for 24 hr. in the presence of a pool of 36 overlapping 20 amino acid residue peptides (final concentration, 4µg/mL each) scanning the full length of HPV 16 E2. Interferon gamma was captured on the substrate by monoclonal antibody (mAb) R4-6A2 (Pharmagin), and detected with biotinylated mAb XMG1.2 (Pharmagin) and a strepavidin-alkaline phosphatase conjugate (Pharmagin). Results are shown in Table A, below. The immunized mice developed CD4⁺ immune responses to HPV (Table A, below).

Immunization with E2 DNA did not induce detectable anti-E2 antibody responses.

**Table A**

| Dose Group | Immunization | E2-specific spots (per 10⁶ cells) |
|---|---|---|
| 1 | E2 DNA 1 | 392 |
| 2 | E2 DNA 2 | 96 |
| 3 | E2 DNA 3 | 134 |
| 4 | Control DNA 1 | 0 |
| 5 | Control DNA 2 | 2 |

### Protection from challenge with JCL-031 cells.

BALB/c mice, immunized with V1Jns:E2 DNA, or control DNA, were challenged by subcutaneous injection of 5 X10⁵ JCL-031 cells into the left inguinal region. Tumor growth was monitored by palpation or caliper measurement for a four-week period. FIGURE 26 reports the fraction of each dose group that remained tumor free. The group that had been immunized with an E2-expressing plasmid was significantly protected from tumor development compared to the control group.

## Claims

1. A synthetic polynucleotide comprising a sequence encoding a human papillomavirus (HPV) protein, the polynucleotide sequence comprising codons optimized for expression in a human host.

2. A polynucleotide according to Claim 1 wherein the protein is selected from the group consisting of: L1, L2, E1, E2, E4, E5, E6 and E7.

3. A polynucleotide according to Claim 2 wherein the protein is selected from the group consisting of: L1, E1, E2, and E7.

4. A polynucleotide according to Claim 2 which is DNA.

5. A polynucleotide according to Claim 4 wherein the protein is L1 and is from an HPV selected from the group consisting of: HPV6a, HPV6b, HPV11, HPV16, HPV18, HPV31, HPV33, HPV35, HPV39, HPV45, HPV51, HPV52, HPV56, HPV58, and HPV68.

6. A polynucleotide according to Claim 5 wherein the protein is an HPV16 L1 protein.

7. A polynucleotide according to Claim 6 which comprises the polynucleotide of SEQ.ID.NO: 1.

8. A polynucleotide according to Claim 4 wherein the protein is an E1 protein and is from an HPV selected from the group consisting of: HPV6a, HPV6b, HPV11, HPV16, HPV18, HPV31, HPV33, HPV35, HPV39, HPV45, HPV51, HPV52, HPV56, HPV58, and HPV 68.

9. A polynucleotide according to Claim 8 which is an HPV16 E1 protein.

10. A polynucleotide according to Claim 9 which comprises the polynucleotide of SEQ. ID.NO:2.

11. A polynucleotide according to Claim 4 wherein the protein is E2 protein and is from an HPV selected from the group consisting of: HPV6a, HPV6b, HPV11, HPV16, HPV18, HPV31, HPV33, HPV35, HPV39, HPV45, HPV51, HPV52, HPV56, HPV58, and HPV 68.

12. A polynucleotide according to Claim 11 which comprises the polynucleotide of SEQ. ID.NO: 3.

13. A polynucleotide according to Claim 4 wherein the protein is E7 and is from an HPV selected from the group consisting of: HPV6a, HPV6b, HPV11, HPV16, HPV18, HPV31, HPV33, HPV35, HPV39, HPV45, HPV51, HPV52, HPV56, HPV58, and HPV68.

14. A polynucleotide according to Claim 13 wherein the protein is an HPV6a protein.

15. A polynucleotide according to Claim 14 which comprises the polynucleotide of SEQ. ID.NO:4.

16. An adenoviral vaccine vector comprising an adenoviral genome with a deletion in the E1 region, and an insert in the E1 region, wherein the insert comprises an expression cassette comprising:
A) a polynucleotide encoding an HPV protein selected from the group consisting of L1, E1, E2, and E7 proteins, wherein the polynucleotide is codon-optimized for expression in a human host cell; and
B) a promoter operably linked to the polynucleotide.

17. A vector according to Claim 16 wherein the adenoviral genome also contains a deleted E3 region.

18. A shuttle plasmid vector comprising a plasmid portion and an adenoviral portion, the adenoviral portion comprising: an adenoviral genome with a deletion in the E1 region, and an insert in the E1 region, wherein the insert comprises an expression cassette comprising:
A) a polynucleotide encoding an HPV protein selected from the group consisting of L1, E1, E2, and E7 proteins, wherein the polynucleotide is codon-optimized for expression in a human host cell; and
B) a promoter operably linked to the polynucleotide.

19. A vaccine plasmid comprising a plasmid portion and an expression cassette portion, the expression cassette portion comprising:
A) a polynucleotide encoding an HPV protein selected from the group consisting of L1, E1, E2, and E7 proteins, wherein the polynucleotide is codon-optimized for expression in a human host cell; and
B) a promoter operably linked to the polynucleotide.

20. A plasmid according to Claim 19 wherein the plasmid portion is V1Js.

21. A vaccine which comprises between 1 ng and 100 mg of the polynucleotide of any one of Claims 1 to 15, and a pharmaceutically acceptable carrier.

22. A vaccine which comprises between 10¹¹-10¹² particles of an adenoviral vector carrying the polynucleotide of any one of Claims 1 to 15 and a pharmaceutically acceptable carrier.

23. A vaccine adapted for administration at two time points which comprises:
a first part
A) comprising a first vector comprising a polynucleotide encoding an HPV protein selected from the group consisting of L1, E1, E2, and E7 proteins, wherein the polynucleotide is codon-optimized for expression in a human host cell;
and a second part
B) comprising a second vector comprising adenoviral vaccine vector comprising an adenoviral genome with a deletion in the E1 region, and an insert in the E1 region, wherein the insert comprises an expression cassette comprises
i) a polynucleotide encoding an HPV protein selected from the group consisting of L1, E1, E2, and E7 proteins, wherein the polynucleotide is codon-optimized for expression in a human host cell; and
ii) a promoter operably linked to the polynucleotide.

24. A method of making a HPV protein comprising expressing in a host cell a synthetic polynucleotide encoding a human papillomavirus (HPV) protein, the polynucleotide sequence comprising codons optimized for expression in a human host.

## Patentansprüche

1. Synthetisches Polynukleotid, umfassend eine Sequenz, welche für ein Human-Papillomavirus (HPV)-Protein kodiert, wobei die Polynukleotidsequenz Codons umfaßt, die für die Expression in einem humanen Wirt optimiert sind.

2. Polynukleotid nach Anspruch 1, wobei das Protein aus der Gruppe, bestehend aus L1, L2, E1, E2, E4, E5, E6 und E7, ausgewählt ist.

3. Polynukleotid nach Anspruch 2, wobei das Protein aus der Gruppe, bestehend aus L1, E1, E2 und E7, ausgewählt ist.

4. Polynukleotid nach Anspruch 2, welches DNA ist.

5. Polynukleotid nach Anspruch 4, wobei das Protein L1 ist und von einem HPV stammt, das aus der Gruppe, bestehend aus HPV6a, HPV6b, HPV11, HPV16, HPV18, HPV31, HPV33, HPV35, HPV39, HPV45, HPV51, HPV52, HPV56, HPV58 und HPV68, ausgewählt ist.

6. Polynukleotid nach Anspruch 5, wobei das Protein ein HPV16-L1-Protein ist.

7. Polynukleotid nach Anspruch 6, welches das Polynukleotid von SEQ-ID-Nr. 1 umfaßt.

8. Polynukleotid nach Anspruch 4, wobei das Protein ein E1-Protein ist und von einem HPV stammt, das aus der Gruppe, bestehend aus HPV6a, HPV6b, HPV11, HPV16, HPV18, HPV31, HPV33, HPV35, HPV39, HPV45, HPV51, HPV52, HPV56, HPV58 und HPV68, ausgewählt ist.

9. Polynukleotid nach Anspruch 8, wobei es ein HPV16-E1-Protein ist.

10. Polynukleotid nach Anspruch 9, welches das Polynukleotid von SEQ-ID-Nr. 2 umfaßt.

11. Polynukleotid nach Anspruch 4, wobei das Protein ein E2-Protein ist und von einem HPV stammt, das aus der Gruppe, bestehend aus HPV6a, HPV6b, HPV11, HPV16, HPV18, HPV31, HPV33, HPV35, HPV39, HPV45, HPV51, HPV52, HPV56, HPV58 und HPV68, ausgewählt ist.

12. Polynukleotid nach Anspruch 11, welches das Polynukleotid von SEQ-ID-Nr. 3 umfaßt.

13. Polynukleotid nach Anspruch 4, wobei das Protein E7 ist und von einem HPV stammt, das aus der Gruppe, bestehend aus HPV6a, HPV6b, HPV11, HPV16, HPV18, HPV31, HPV33, HPV35, HPV39, HPV45, HPV51, HPV52, HPV56, HPV58 und HPV68, ausgewählt ist.

14. Polynukleotid nach Anspruch 13, wobei das Protein ein HPV6a-Protein ist.

15. Polynukleotid nach Anspruch 14, welches das Polynukleotid von SEQ-ID-Nr. 4 umfaßt.

16. Adenovirus-Vakzin-Vektor, umfassend ein adenovirales Genom mit einer Deletion in der E1-Region und einer Insertion in der E1-Region, wobei die Insertion eine Expressionskassette umfaßt, umfassend
A) ein Polynukleotid, kodierend für ein HPV-Protein, das aus der Gruppe, bestehend aus L1-, E1-, E2- und E7-Proteinen, ausgewählt ist, wobei das Polynukleotid für die Expression in einer humanen Wirtszelle codon-optimiert ist, und
B) einen Promotor in funktionsfähiger Verknüpfung mit dem Polynukleotid.

17. Vektor nach Anspruch 16, wobei das adenovirale Genom auch eine deletierte E3-Region enthält.

18. Shuttle-Plasmidvektor, umfassend einen Plasmidanteil und einen adenoviralen Anteil, wobei der adenovirale Anteil ein adenovirales Genom mit einer Deletion in der E1-Region und einer Insertion in der E1-Region umfaßt, wobei die Insertion eine Expressionskassette umfaßt, umfassend
A) ein Polynukleotid, kodierend für ein HPV-Protein, das aus der Gruppe, bestehend aus L1-, E1-, E2- und E7-Proteinen, ausgewählt ist, wobei das Polynukleotid für die Expression in einer humanen Wirtszelle codon-optimiert ist, und
B) einen Promotor in funktionsfähiger Verknüpfung mit dem Polynukleotid.

19. Vakzin-Plasmid, umfassend einen Plasmidanteil und einen Expressionskassettenanteil, wobei der Expressionskassettenanteil umfaßt
A) ein Polynukleotid, kodierend für ein HPV-Protein, das aus der Gruppe, bestehend aus L1-, E1-, E2- und E7-Proteinen, ausgewählt ist, wobei das Polynukleotid für die Expression in einer humanen Wirtszelle codon-optimiert ist, und
B) einen Promotor in funktionsfähiger Verknüpfung mit dem Polynukleotid.

20. Plasmid nach Anspruch 19, wobei der Plasmidanteil V1Js ist.

21. Vakzin, welches zwischen 1 ng und 100 mg des Polynukleotids nach irgendeinem der Ansprüche 1 bis 15 und einen pharmazeutisch annehmbaren Träger umfaßt.

22. Vakzin, welches zwischen 10¹¹ - 10¹² Partikel eines adenoviralen Vektors, der das Polynukleotid nach irgendeinem der Ansprüche 1 bis 15 trägt, und einen pharmazeutisch annehmbaren Träger umfaßt.

23. Vakzin, das für die Verabreichung zu zwei Zeitpunkten adaptiert ist, welches umfaßt:
einen ersten Teil
A) umfassend einen ersten Vektor, der ein Polynukleotid umfaßt, kodierend für ein HPV-Protein, das aus der Gruppe, bestehend aus L1-, E1-, E2- und E7-Proteinen, ausgewählt ist, wobei das Polynukleotid für die Expression in einer humanen Wirtszelle codon-optimiert ist, und einen zweiten Teil
B) umfassend einen zweiten Vektor, der einen adenoviralen Vakzin-Vektor umfaßt, welcher ein adenovirales Genom mit einer Deletion in der E1-Region und einer Insertion in der E1-Region umfaßt, wobei die Insertion eine Expressionskassette umfaßt, umfassend
i) ein Polynukleotid, kodierend für ein HPV-Protein, das aus der Gruppe, bestehend aus L1-, E1-, E2- und E7-Proteinen, ausgewählt ist, wobei das Polynukleotid für die Expression in einer humanen Wirtszelle codon-optimiert ist, und
ii) einen Promotor in funktionsfähiger Verknüpfung mit dem Polynukleotid.

24. Verfahren zur Herstellung eines HPV-Proteins, umfassend das Exprimieren eines synthetischen Polynukleotids, das für ein Human-Papillomavirus (HPV)-Protein kodiert, in einer Wirtszelle, wobei die Polynukleotidsequenz Codons umfaßt, die für die Expression in einem humanen Wirt optimiert sind.

## Revendications

1. Polynucléotide synthétique comprenant une séquence codant pour une protéine du papillomavirus humain (HPV), la séquence de polynucléotide comprenant des codons optimisés pour l'expression chez un hôte humain.

2. Polynucléotide selon la revendication 1, dans lequel la protéine est choisie dans le groupe constitué par : L1, L2, E1, E2, E4, E5, E6 et E7.

3. Polynucléotide selon la revendication 2, dans lequel la protéine est choisie dans le groupe constitué par : L1, E1, E2 et E7.

4. Polynucléotide selon la revendication 2, qui est un ADN.

5. Polynucléotide selon la revendication 4, dans lequel la protéine est L1, et provient d'un HPV choisi dans le groupe constitué par : HPV6a, HPV6b, HPV11, HPV16, HPV18, HPV31, HPV33, HPV35, HPV39, HPV45, HPV51, HPV52, HPV56, HPV58 et HPV68.

6. Polynucléotide selon la revendication 5, dans lequel la protéine est une protéine L1 de HPV16.

7. Polynucléotide selon la revendication 6, qui comprend le polynucléotide de la SEQ ID n° : 1.

8. Polynucléotide selon la revendication 4, dans lequel la protéine est une protéine E1, et provient d'un HPV choisi dans le groupe constitué par : HPV6a, HPV6b, HPV11, HPV16, HPV18, HPV31, HPV33, HPV35, HPV39, HPV45, HPV51, HPV52, HPV56, HPV58 et HPV68.

9. Polynucléotide selon la revendication 8, qui est une protéine E1 de HPV16.

10. Polynucléotide selon la revendication 9, qui comprend le polynucléotide de la SEQ ID n° : 2.

11. Polynucléotide selon la revendication 4, dans lequel la protéine est une protéine E2, et provient d'un HPV choisi dans le groupe constitué par : HPV6a, HPV6b, HPV11, HPV16, HPV18, HPV31, HPV33, HPV35, HPV39, HPV45, HPV51, HPV52, HPV56, HPV58 et HPV68.

12. Polynucléotide selon la revendication 11, qui comprend le polynucléotide de la SEQ ID n° : 3.

13. Polynucléotide selon la revendication 4, dans lequel la protéine est E7, et provient d'un HPV choisi dans le groupe constitué par : HPV6a, HPV6b, HPV11, HPV16, HPV18, HPV31, HPV33, HPV35, HPV39, HPV45, HPV51, HPV52, HPV56, HPV58 et HPV68.

14. Polynucléotide selon la revendication 13, dans lequel la protéine est une protéine de HPV6a.

15. Polynucléotide selon la revendication 14, qui comprend le polynucléotide de la SEQ ID n° : 4.

16. Vecteur de vaccin d'adénovirus comprenant un génome d'adénovirus avec une délétion dans la région E1, et un insert dans la région E1, dans lequel l'insert comprend une cassette d'expression comprenant :
A) un polynucléotide codant pour une protéine de HPV choisie dans le groupe constitué par les protéines L1, E1, E2 et E7, dans lequel le polynucléotide a des codons optimisés pour l'expression chez une cellule hôte humaine ; et
B) un promoteur lié de façon opérationnelle au polynucléotide.

17. Vecteur selon la revendication 16, dans lequel le génome d'adénovirus contient aussi une région E3 délétée.

18. Vecteur plasmidique navette comprenant une partie de plasmide et une partie d'adénovirus, la partie d'adénovirus comprenant un génome d'adénovirus avec une délétion dans la région E1, et un insert dans la région E1, dans lequel l'insert comprend une cassette d'expression comprenant :
A) un polynucléotide codant pour une protéine de HPV choisie dans le groupe constitué par les protéines L1, E1, E2 et E7, dans lequel le polynucléotide a des codons optimisés pour l'expression chez une cellule hôte humaine ; et
B) un promoteur lié de façon opérationnelle au polynucléotide.

19. Vaccin plasmidique comprenant une partie de plasmide et une partie de cassette d'expression, la partie de cassette d'expression comprenant :
A) un polynucléotide codant pour une protéine de HPV choisie dans le groupe constitué par les protéines L1, E1, E2 et E7, dans lequel le polynucléotide a des codons optimisés pour l'expression chez une cellule hôte humaine ; et
B) un promoteur lié de façon opérationnelle au polynucléotide.

20. Plasmide selon la revendication 19, dans lequel la partie plasmidique est V1Js.

21. Vaccin qui comprend entre 1 ng et 100 mg du polynucléotide selon l'une quelconque des revendications 1 à 15 et un vecteur pharmaceutiquement acceptable.

22. Vaccin qui comprend entre 10¹¹ et 10¹² particules d'un vecteur d'adénovirus portant le polynucléotide selon l'une quelconque des revendications 1 à 15 et un vecteur pharmaceutiquement acceptable.

23. Vaccin adapté à l'administration à deux points de temps, qui comprend :
une première partie
A) comprenant un premier vecteur comprenant un polynucléotide codant pour une protéine de HPV choisie dans le groupe constitué par les protéines L1, E1, E2 et E7, dans lequel le polynucléotide a des codons optimisés pour l'expression dans une cellule hôte humaine ;
et une seconde partie
B) comprenant un second vecteur comprenant un vecteur de vaccin d'adénovirus contenant un génome d'adénovirus avec une délétion dans la région E1, et un insert dans la région E1, dans lequel l'insert comprend une cassette d'expression qui contient :
i) un polynucléotide codant pour une protéine de HPV choisie dans le groupe constitué par les protéines L1, E1, E2 et E7, dans lequel le polynucléotide a des codons optimisés pour l'expression chez une cellule hôte humaine ; et
ii) un promoteur lié de façon opérationnelle au polynucléotide.

24. Procédé de fabrication d'une protéine de HPV comprenant l'expression dans une cellule hôte d'un polynucléotide synthétique codant pour une protéine de papillomavirus humain (HPV), la séquence de polynucléotide comprenant des codons optimisés pour l'expression chez un hôte humain.


### SEQUENCE LISTING

<110> Merck & Co., Inc.
<120> SYNTHETIC HUMAN PAPILLOMAVIRUS GENES
<130> 20413Y PCT
<150> 60/150,728
   <151> 1999-08-25
<150> 60/210,143
   <151> 2000-06-07
<160> 150
<170> FastSEQ for Windows Version 4.0
<210> 1
   <211> 1518
   <212> DNA
   <213> Artificial Sequence
<220>
   <223> Codon-Optimized HPV16 L1
<400> 1
   atgagcctgt ggctgcccag cgaggccacc gtgtacctgc ctcccgtgcc cgtgagcaag      60
   gtggtgagca ccgacgagta cgtggcccgc accaacatct actaccacgc cggcaccagc     120
   cgcctgctgg ccgtgggcca cccctacttc cccatcaaga agcccaacaa caacaagatc     180
   ctggtgccca aggtgagcgg cctgcagtac cgcgtgttcc gcatccacct gcccgacccc     240
   aacaagttcg gcttccccga cacaagcttc tacaaccccg acacccagcg cctggtgtgg     300
   gcctgcgtgg gcgtggaggt gggccgcggc cagcccctgg gcgtgggcat cagcggccac     360
   cccctgctga acaagctgga cgacaccgag aacgccagcg cctacgccgc caacgccggc     420
   gtggacaacc gcgagtgcat cagcatggac tacaagcaga cccagctgtg cctgatcggc 480
   tgcaagcctc ccatcggcga gcactggggc aagggcagcc cctgcaccaa cgtggccgtg 540
   aaccccggcg actgccctcc cctggagctg atcaacaccg tgatccagga cggcgacatg 600
   gtggacaccg gcttcggcgc catggacttc accaccctgc aggccaacaa gagcgaggtg 660
   cccctggaca tctgcaccag catctgcaag taccccgact acatcaagat ggtgagcgag 720
   ccctacggcg acagcctgtt cttctacctg cgccgcgagc agatgttcgt gcgccacctg 780
   ttcaaccgcg ccggcgccgt gggcgagaac gtgcccgacg acctgtacat caagggcagc 840
   ggcagcaccg ccaacctggc cagcagcaac tacttcccca ctcccagcgg cagcatggtg 900
   accagcgacg cccaaatctt caacaagccc tactggctgc agcgcgccca gggccacaac 960
   aacggcatct gctggggcaa ccagctgttc gtgaccgtgg tggacaccac ccgcagcacc 1020
   aacatgagcc tgtgcgccgc catcagcacc agcgagacca cctacaagaa caccaacttc 1080
   aaggagtacc tgcgccacgg cgaggagtac gacctgcagt tcatcttcca gctgtgcaag 1140
   atcaccctga ccgccgacgt gatgacctac atccacagca tgaacagcac catcctggag 1200
   gactggaact tcggcctgca gccccctccc ggcggtaccc tggaggacac ctaccgcttc 1260
   gtgaccagcc aggccatcgc ctgccagaag cacacccctc ccgctcccaa ggaggatccc 1320
   ctgaagaagt acaccttctg ggaggtgaac ctgaaggaga agttcagcgc cgacctggac 1380
   cagttccccc tgggccgcaa gttcctgctg caggccggcc tgaaggccaa gcccaagttc 1440
   accctgggca agcgcaaggc cacccccacc accagcagca ccagcaccac cgccaagcgc 1500
   aagaagcgca agctgtaa 1518
<210> 2
   <211> 1950
   <212> DNA
   <213> Artificial Sequence
<220>
   <223> Mutant, Codon-Optimized HPV16 E1
<400> 2
   atggccgacc ccgccggcac caacggcgag gagggcaccg gctgcaacgg ctggttctac 60
   gtggaggccg tggtggagaa gaagaccggc gacgccatca gcgacgacga gaacgagaac 120
   gacagcgaca ccggcgagga cctggtggac ttcatcgtga acgacaacga ctacctgacc 180
   caggccgaga ccgagaccgc ccacgccctg ttcaccgccc aggaggccaa gcagcaccgc 240
   gacgccgtgc aggtgctgaa gcgcaagtac ctgggcagcc ccctgagcga catcagcggc 300
   tgcgtcgaca acaacatcag cccccgcctg aaggccatct gcatcgagaa gcagagccgc 360
   gccgccaagc gccgcctgtt cgagagcgag gacagcggct acggcaacac cgaggtggag 420
   acccagcaga tgctgcaggt ggagggccgc cacgagaccg agaccccctg cagccagtac 480
   agcggcggca gcggcggcgg ctgcagccag tacagcagcg gcagcggcgg cgagggcgtg 540
   agcgagcgcc acaccatctg ccagacccct ctgaccaaca tcctgaacgt gctgaagacc 600
   agcaacgcca aggccgccat gctggccaag ttcaaggagc tgtacggcgt gagcttcagc 660
   gagctggtgc gccccttcaa gagcaacaag agcacctgct gcgactggtg catcgccgcc 720
   ttcggcctga cccccagcat cgccgacagc atcaagaccc tgctgcagca gtactgcctg 780
   tacctgcaca tccagagcct ggcctgcagc tggggcatgg tggtgctgct gctggtgcgc 840
   tacaagtgcg gcaagaaccg cgagaccatc gagaagctgc tgagcaagct gctgtgcgtg 900
   agccccatgt gcatgatgat cgagcctccc aagcttcgca gcaccgccgc cgccctgtac 960
   tggtacaaga ccggcatcag caacatcagc gaggtgtacg gcgacacccc cgagtggatc 1020
   cagcgccaga ccgtgctgca gcacagcttc aacgactgca ccttcgagct gagccagatg 1080
   gtgcagtggg cctacgacaa cgacatcgtg gacgacagcg agatcgccta caagtacgcc 1140
   cagctggccg acaccaacag caacgccagc gccttcctga agagcaacag ccaggccaag 1200
   atcgtgaagg actgcgccac catgtgccgc cactacaagc gcgccgagaa gaagcagatg 1260
   agcatgagcc agtggatcaa gtaccgctgc gaccgcgtgg acgacggcgg cgaccgcaag 1320
   cagatcgtga tgttcctgcg ctaccagggc gtggaattca tgagcttcct gaccgccctg 1380
   aagcgcttcc tgcagggcat ccccaagaag aactgcatcc tgctgtacgg cgccgccaac 1440
   accgacaaga gcctgttcgg catgagcctg atgaagttcc tgcagggcag cgtgatctgc 1500
   ttcgtgaaca gcaagagcca cttctggctg cagcccctgg ccgacgccaa gatcggcatg 1560
   ctggacgacg ccaccgtgcc ctgctggaac tacatcgacg acaacctgcg caacgccctg 1620
   gacggcaacc tggtgagcat ggacgtgaag caccgccccc tggtgcagct gaagtgccct 1680
   cccctgctga tcaccagcaa catcaacgcc ggcaccgaca gccgctggcc ctacctgcac 1740
   aaccgcctgg tggtgttcac cttccccaac gagttcccct tcgacgagaa cggtaacccc 1800
   gtgtacgagc tgaacgacaa gaactggaag agcttcttca gccgcacctg gagccgcctg 1860
   agcctgcacg aggacgagga caaggagaac gacggcgaca gcctgcccac cttcaagtgc 1920
   gtgagcggcc agaacaccaa caccctgtaa 1950
<210> 3
   <211> 1098
   <212> DNA
   <213> Artificial Sequence
<220>
   <223> Mutant, Codon-Optimized HPV16 E2
<400> 3
   atggagaccc tgtgccagcg cctgaacgtg tgccaggaca agatcctgac ccactacgag      60
   aacgacagca ccgacctgcg cgaccacatc gactactgga agcacatgcg cctggcctgc     120
   gccatctact acaaggcccg cgagatgggc ttcaagcaca tcaaccacca ggtggtgccc     180
   accctggccg tgagcaagaa caaggccctg caggccgccg agctgcagct gaccctggag     240
   accatctaca acagccagta cagcaacgag aagtggaccc tgcaggacgt gagcctggag     300
   gtgtacctga ccgcccccac cggctgcatc aagaagcacg gctacaccgt ggaggtgcag     360
   ttcgacggcg acatctgcaa caccatgcac tacaccaact ggacccacat ctacatctgc     420
   gaggaggcca gcgtgaccgt ggtggagggc caggtggact actacggcct gtactacgtg     480
   cacgagggca tccgcaccta cttcgtgcag ttcaaggacg acgccgagaa gtacagcaag     540
   aacaaggtgt gggaggtgca cgccggcggc caggtgatcc tgtgccccac cagcgtgttc     600
   agcagcaacg aggtgagcag ccccgagacc atccgccagc acctggccaa ccacagcgcc     660
   gccacccaca ccaaggccgt ggccctgggc accgaggaga cccagaccac catccagcgc     720
   ccccgcagcg agcccgacac cggcaacccc tgccacacca ccaagctgct gcaccgcgac     780
   agcgtggaca gcgcccccat cctgaccgcc ttcaacagca gccacaaggg ccgcatcaac     840
   tgcaacagca acaccacccc catcgtgcac ctgaagggcg acgccaacac cctgaagtgc     900
   ctgcgctacc gcttcaagaa gcactgcaag ctgtacaccg ccgtgagcag cacctggcac     960
   tggaccggcc acaacgtgaa gcacaagagc gccatcgtga ccctgaccta cgacagcgag    1020
   tggcagcgcg accagttcct gagccaggtg aagatcccca agaccatcac cgtgagcacc    1080
   ggcttcatga gcatctaa                                                   1098
<210> 4
   <211> 297
   <212> DNA
   <213> Artificial Sequence
<220>
   <223> Mutant, Codon-Optimized HPV16 E7
<400> 4
   atgcacggcg acacccccac cctgcacgag tacatgctgg acctgcagcc cgagaccacc      60
   gacctgtacg gctacggcca gctgaacgac agcagcgagg aggaggacga gatcgacggc     120
   cccgccggcc aggccgagcc cgaccgcgcc cactacaaca tcgtgacctt ctgctgcaag     180
   tgcgacagca ccctgcgcct gtgcgtgcag agcacccacg tggacatccg caccctggag     240
   gacctgctga tgggcaccct gggcatcgtg tgccccatct gcagccagaa gccctaa        297
<210> 5
   <211> 297
   <212> DNA
   <213> Artificial Sequence
<220>
   <223> Codon-Optimized HPV6a E7
<400> 5
   atgcacggcc gccacgtgac cctgaaggac atcgtgctgg acctgcagcc tcccgacccc 60
   gtgggcctgc actgctacga gcagctggtg gacagcagcg aggacgaggt ggacgaggtg 120
   gacggccagg acagccagcc cctgaagcag cacttccaga tcgtgacctg ctgctgcggc 180
   tgcgacagca acgtgcgcct ggtggtgcag tgcaccgaga ccgacatccg cgaggtgcag 240
   cagctcctgc tgggtaccct gaacatcgtg tgccccatct gcgctcccaa gacctaa 297
<210> 6
   <211> 318
   <212> DNA
   <213> Artificial Sequence
<220>
   <223> Codon-Optimized HPV18 E7
<400> 6
   atgcacggcc ccaaggccac cctgcaggac atcgtgctgc acctggagcc ccagaacgag 60
   atccccgtgg acctgctgtg ccacgagcag ctgagcgaca gcgaggagga gaacgacgag 120
   atcgacggcg tgaaccacca gcacctgccc gctcgcaggg ccgagcccca gcgccacacc 180
   atgctgtgca tgtgctgcaa gtgcgaggcc cgcatcgagc tggtggtgga gagcagcgct 240
   gacgacctgc gcgccttcca gcagctgttc ctgaacaccc tgagcttcgt gtgcccctgg 300
   tgcgccagcc agcagtaa 318
<210> 7
   <211> 1107
   <212> DNA
   <213> Artificial Sequence
<220>
   <223> Codon-Optimized HPV6a E2
<400> 7
   atggaggcca tcgccaagcg cctggacgcc tgccaggagc agctgctgga gctgtacgag 60
   gagaacagca ccgacctgca caagcacgtg ctgcactgga agtgcatgcg ccacgagagc 120
   gtgctgctgt acaaggccaa gcagatgggc ctgagccaca tcggcatgca ggtggtgcct 180
   cctctgaagg tgagcgaggc caagggccac aacgccatcg agatgcagat gcacctcgag 240
   agcctgctgc gcaccgagta cagcatggag ccctggaccc tgcaggagac cagctacgag 300
   atgtggcaga cccctcccaa gcgctgcttc aagaagcgcg gcaagaccgt ggaggtgaag 360
   ttcgacggct gcgccaacaa caccatggac tacgtggtgt ggaccgacgt gtacgtgcag 420
   gacaacgaca cctgggtgaa ggtgcacagc atggtggacg ccaagggcat ctactacacc 480
   tgtggccagt tcaagaccta ctacgtgaac ttcgtgaagg aggccgagaa gtacggcagc 540
   accaagcact gggaggtgtg ctacggcagc accgtgatct gcagccccgc tagcgtgagc 600
   agcaccaccc aggaggtgag catccccgag agcaccacct acactcccgc ccagaccagc 660
   accctggtga gcagcagcac caaggaggac gccgtgcaga cccctcctcg caagcgcgcc 720
   cgcggcgtgc agcagagccc ctgcaacgcc ctgtgcgtgg cccacatcgg ccccgtggat 780
   agcggcaacc acaacctgat caccaacaac cacgaccagc accagcgccg caacaacagc 840
   aacagcagcg ccactcccat cgtgcagttc cagggcgaga gcaactgcct gaagtgcttc 900
   cgctaccgcc tgaacgatcg ccaccgccac ctgttcgacc tgatcagcag cacctggcac 960
   tgggccagca gcaaggctcc ccacaagcac gccatcgtga ccgtgaccta cgacagcgag 1020
   gagcagcgcc agcagttcct ggacgtggtg aagatccctc ccaccatcag ccacaagctg 1080
   ggcttcatga gcctgcacct gctgtaa 1107
<210> 8
   <211> 1098
   <212> DNA
   <213> Artificial Sequence
<220>
   <223> Codon-Optimized HPV18 E2
<400> 8
   atgcagactc ccaaggagac cctgagcgag cgcctgagcg ccctgcagga caagatcatc      60
   gaccactacg agaacgacag caaggacatc gacagccaga tccagtactg gcagctgatc     120
   cgctgggaga acgccatctt cttcgccgct cgcgagcacg ggatccagac cctgaaccac     180
   caggtggtgc ccgcctacaa catcagcaag agcaaggccc acaaggccat cgagctgcag     240
   atggccctgc agggcctggc ccagagcgcc tacaagaccg aggactggac cctgcaggac     300
   acctgcgagg agctgtggaa caccgagccc acccactgct tcaagaaggg aggccagacc     360
   gtgcaggtgt acttcgacgg caacaaggac aactgcatga actacgtggc ctgggacagc     420
   gtgtactaca tgaccgacgc cggcacctgg gacaagaccg ccacctgcgt gagccaccgc     480
   ggcctgtact acgtgaagga gggctacaac accttctaca tcgagttcaa gagcgagtgc     540
   gagaagtacg gcaacaccgg cacctgggag gtgcacttcg gcaacaacgt gatcgactgc     600
   aacgacagca tgtgcagcac cagcgacgac accgtgagcg ccacccagct ggtgaagcag     660
   ctgcagcaca ctcccagccc ctacagcagc accgtgagcg tgggcaccgc caagacctac     720
   ggccagacca gcgccgccac tcgccctggc cactgcggcc tggccgagaa gcagcactgc     780
   gggcccgtga accctctgct gggcgccgcc accgccaccg gcaacaacaa gcgccgcaag     840
   ctgtgcagcg gcaacaccac tcccatcatc cacctgaagg gcgaccgcaa cagcctgaag     900
   tgcctgcggt accgcctgcg caagcacagc gaccactacc gcgacatcag cagcacctgg     960
   cactggaccg gcgccgggaa cgagaagacc ggcatcctga ccgtgaccta ccacagcgag    1020
   acccagcgca ccaagttcct gaacaccgtg gccatccccg acagcgtgca gatcctggtg    1080
   ggctacatga ccatgtaa                                                  1098
<210> 9
   <211> 129
   <212> DNA
   <213> Artificial Sequence
<220>
   <223> Codon-Optimized HPV16 L1 fragment
<400> 9
   atgagcctgt ggctgcccag cgaggccacc gtgtacctgc ctcccgtgcc cgtgagcaag      60
   gtggtgagca ccgacgagta cgtggcccgc accaacatct actaccacgc cggcaccagc     120
   cgcctgctg                                                             129
<210> 10
   <211> 129
   <212> DNA
   <213> Artificial Sequence
<220>
   <223> Codon-Optimized HPV16 L1 fragment
<400> 10
   cgcatccacc tgcccgaccc caacaagttc ggcttccccg acacaagctt ctacaacccc 60
   gacacccagc gcctggtgtg ggcctgcgtg ggcgtggagg tgggccgcgg ccagcccctg 120
   ggcgtgggc 129
<210> 11
   <211> 129
   <212> DNA
   <213> Artificial Sequence
<220>
   <223> Codon-Optimized HPV16 L1 fragment
<400> 11
   gagtgcatca gcatggacta caagcagacc cagctgtgcc tgatcggctg caagcctccc 60
   atcggcgagc actggggcaa gggcagcccc tgcaccaacg tggccgtgaa ccccggcgac 120
   tgccctccc 129
<210> 12
   <211> 132
   <212> DNA
   <213> Artificial Sequence
<220>
   <223> Codon-Optimized HPV16 L1 fragment
<400> 12
   gccaacaaga gcgaggtgcc cctggacatc tgcaccagca tctgcaagta ccccgactac 60
   atcaagatgg tgagcgagcc ctacggcgac agcctgttct tctacctgcg ccgcgagcag 120
   atgttcgtgc gc 132
<210> 13
   <211> 129
   <212> DNA
   <213> Artificial Sequence
<220>
   <223> Codon-Optimized HPV16 L1 fragment
<400> 13
   gccagcagca actacttccc cactcccagc ggcagcatgg tgaccagcga cgcccaaatc 60
   ttcaacaagc cctactggct gcagcgcgcc cagggccaca acaacggcat ctgctggggc 120
   aaccagctg 129
<210> 14
   <211> 135
   <212> DNA
   <213> Artificial Sequence
<220>
   <223> Codon-Optimized HPV16 L1 fragment
<400> 14
   gagtacctgc gccacggcga ggagtacgac ctgcagttca tcttccagct gtgcaagatc      60
   accctgaccg ccgacgtgat gacctacatc cacagcatga acagcaccat cctggaggac     120
   tggaacttcg gcctg                                                      135
<210> 15
   <211> 135
   <212> DNA
   <213> Artificial Sequence
<220>
   <223> Codon-Optimized HPV16 L1 fragment
<400> 15
   gctcccaagg aggatcccct gaagaagtac accttctggg aggtgaacct gaaggagaag      60
   ttcagcgccg acctggacca gttccccctg ggccgcaagt tcctgctgca ggccggcctg     120
   aaggccaagc ccaag                                                      135
<210> 16
   <211> 135
   <212> DNA
   <213> Artificial Sequence
<220>
   <223> Codon-Optimized HPV16 L1 fragment
<400> 16
   gttggggtcg ggcaggtgga tgcggaacac gcggtactgc aggccgctca ccttgggcac      60
   caggatcttg ttgttgttgg gcttcttgat ggggaagtag gggtggccca cggccagcag     120
   gcggctggtg ccggc                                                      135
<210> 17
   <211> 132
   <212> DNA
   <213> Artificial Sequence
<220>
   <223> Codon-Optimized HPV16 L1 fragment
<400> 17
   cttgtagtcc atgctgatgc actcgcggtt gtccacgccg gcgttggcgg cgtaggcgct      60
   ggcgttctcg gtgtcgtcca gcttgttcag cagggggtgg ccgctgatgc ccacgcccag    120
   gggctggccg cg                                                         132
<210> 18
   <211> 129
   <212> DNA
   <213> Artificial Sequence
<220>
   <223> Codon-Optimized HPV16 L1 fragment
<400> 18
   caggggcacc tcgctcttgt tggcctgcag ggtggtgaag tccatggcgc cgaagccggt      60
   gtccaccatg tcgccgtcct ggatcacggt gttgatcagc tccaggggag ggcagtcgcc     120
   ggggttcac                                                             129
<210> 19
   <211> 132
   <212> DNA
   <213> Artificial Sequence
<220>
   <223> Codon-Optimized HPV16 L1 fragment
<400> 19
   gggagtgggg aagtagttgc tgctggccag gttggcggtg ctgccgctgc ccttgatgta      60
   caggtcgtcg ggcacgttct cgcccacggc gccggcgcgg ttgaacaggt ggcgcacgaa     120
   catctgctcg cg                                                         132
<210> 20
<211> 144
   <212> DNA
   <213> Artificial Sequence
<220>
   <223> Codon-Optimized HPV16 L1 fragment
<400> 20
   ctcctcgccg tggcgcaggt actccttgaa gttggtgttc ttgtaggtgg tctcgctggt 60
   gctgatggcg gcgcacaggc tcatgttggt gctgcgggtg gtgtccacca cggtcacgaa 120
   cagctggttg ccccagcaga tgcc 144
<210> 21
   <211> 132
   <212> DNA
   <213> Artificial Sequence
<220>
   <223> Codon-Optimized HPV16 L1 fragment
<400> 21
   cttcagggga tcctccttgg gagcgggagg ggtgtgcttc tggcaggcga tggcctggct 60
   ggtcacgaag cggtaggtgt cctccagggt accgccggga gggggctgca ggccgaagtt 120
   ccagtcctcc ag 132
<210> 22
   <211> 123
   <212> DNA
   <213> Artificial Sequence
<220>
   <223> Codon-Optimized HPV16 L1 fragment
<400> 22
   cactagagat ctgaattctt acagcttgcg cttcttgcgc ttggcggtgg tgctggtgct 60
   gctggtggtg ggggtggcct tgcgcttgcc cagggtgaac ttgggcttgg ccttcaggcc 120
   ggc 123
<210> 23
   <211> 21
   <212> DNA
   <213> Artificial Sequence
<220>
   <223> Codon-Optimized HPV16 L1 fragment
<400> 23
   cgcggccagc ccctgggcgt g 21
<210> 24
   <211> 24
   <212> DNA
   <213> Artificial Sequence
<220>
   <223> Codon-Optimized HPV16 L1 fragment
<400> 24
   gcccacgccc aggggctggc cgcg 24
<210> 25
   <211> 21
   <212> DNA
   <213> Artificial Sequence
<220>
   <223> Codon-Optimized HPV16 L1 fragment
<400> 25
   gccaacaaga gcgaggtgcc c 21
<210> 26
   <211> 24
   <212> DNA
   <213> Artificial Sequence
<220>
   <223> Codon-Optimized HPV16 L1 fragment
<400> 26
   caggggcacc tcgctcttgt tggc 24
<210> 27
   <211> 23
   <212> DNA
   <213> Artificial Sequence
<220>
   <223> Codon-Optimized HPV16 L1 fragment
<400> 27
   gccagcagca actacttccc cac 23
<210> 28
   <211> 23
   <212> DNA
   <213> Artificial Sequence
<220>
   <223> Codon-Optimized HPV16 L1 fragment
<400> 28
   gggagtgggg aagtagttgc tgc 23
<210> 29
   <211> 24
   <212> DNA
   <213> Artificial Sequence
<220>
   <223> Codon-Optimized HPV16 L1 fragment
<400> 29
   ctggaggact ggaacttcgg cctg 24
<210> 30
   <211> 24
   <212> DNA
   <213> Artificial Sequence
<220>
   <223> Codon-Optimized HPV16 L1 fragment
<400> 30
   caggccgaag ttccagtcct ccag 24
<210> 31
   <211> 25
   <212> DNA
   <213> Artificial Sequence
<220>
   <223> Codon-Optimized HPV16 L1 fragment
<400> 31
   cactagagat ctgaattctt acagc 25
<210> 32
   <211> 38
   <212> DNA
   <213> Artificial Sequence
<220>
   <223> Codon-Optimized HPV16 L1 fragment
<400> 32
   catctcagat ctgccaccat gagcctgtgg ctgcccag 38
<210> 33
   <211> 129
   <212> DNA
   <213> Artificial Sequence
<220>
   <223> Codon-Optimized HPV16 E1 fragment
<400> 33
   atggccgacc ccgccggcac caacggcgag gagggcaccg gctgcaacgg ctggttctac 60
   gtggaggccg tggtggagaa gaagaccggc gacgccatca gcgacgacga gaacgagaac 120
   gacagcgac 129
<210> 34
   <211> 132
   <212> DNA
   <213> Artificial Sequence
<220>
   <223> Codon-Optimized HPV16 E1 fragment
<400> 34
   gtgctgcttg gcctcctggg cggtgaacag ggcgtgggcg gtctcggtct cggcctgggt 60
   caggtagtcg ttgtcgttca cgatgaagtc caccaggtcc tcgccggtgt cgctgtcgtt 120
   ctcgttctcg tc 132
<210> 35
   <211> 132
   <212> DNA
   <213> Artificial Sequence
<220>
   <223> Codon-Optimized HPV16 E1 fragment
<400> 35
   gcccaggagg ccaagcagca ccgcgacgcc gtgcaggtgc tgaagcgcaa gtacctgggc 60
   agccccctga gcgacatcag cggctgcgtc gacaacaaca tcagcccccg cctgaaggcc 120
   atctgcatcg ag 132
<210> 36
   <211> 131
   <212> DNA
   <213> Artificial Sequence
<220>
   <223> Codon-Optimized HPV16 E1 fragment
<400> 36
   ctcgtggcgg ccctccacct gcagcatctg ctgggtctcc acctcggtgt tgccgtagcc      60
   gctgtcctcg ctctcgaaca ggcggcgctt ggcggcgcgg ctctgcttct cgatgcagat     120
   ggccttcagg c                                                          131
<210> 37
   <211> 132
   <212> DNA
   <213> Artificial Sequence
<220>
   <223> Codon-Optimized HPV16 E1 fragment
<400> 37
   caggtggagg gccgccacga gaccgagacc ccctgcagcc agtacagcgg cggcagcggc      60
   ggcggctgca gccagtacag cagcggcagc ggcggcgagg gcgtgagcga gcgccacacc     120
   atctgccaga cc                                                         132
<210> 38
   <211> 135
   <212> DNA
   <213> Artificial Sequence
<220>
   <223> Codon-Optimized HPV16 E1 fragment
<400> 38
   cttgaagggg cgcaccagct cgctgaagct cacgccgtac agctccttga acttggccag      60
   catggcggcc ttggcgttgc tggtcttcag cacgttcagg atgttggtca gaggggtctg     120
   gcagatggtg tggcg                                                      135
<210> 39
   <211> 135
   <212> DNA
   <213> Artificial Sequence
<220>
   <223> Codon-Optimized HPV16 E1 fragment
<400> 39
   gagctggtgc gccccttcaa gagcaacaag agcacctgct gcgactggtg catcgccgcc 60
   ttcggcctga cccccagcat cgccgacagc atcaagaccc tgctgcagca gtactgcctg 120
   tacctgcaca tccag 135
<210> 40
   <211> 136
   <212> DNA
   <213> Artificial Sequence
<220>
   <223> Codon-Optimized HPV16 E1 fragment
<400> 40
   catggggctc acgcacagca gcttgctcag cagcttctcg atggtctcgc ggttcttgcc 60
   gcacttgtag cgcaccagca gcagcaccac catgccccag ctgcaggcca ggctctggat 120
   gtgcaggtac aggcag 136
<210> 41
   <211> 132
   <212> DNA
   <213> Artificial Sequence
<220>
   <223> Codon-Optimized HPV16 E1 fragment
<400> 41
   ctgctgtgcg tgagccccat gtgcatgatg atcgagcctc ccaagcttcg cagcaccgcc 60
   gccgccctgt actggtacaa gaccggcatc agcaacatca gcgaggtgta cggcgacacc 120
   cccgagtgga tc 132
<210> 42
   <211> 129
   <212> DNA
   <213> Artificial Sequence
<220>
   <223> Codon-Optimized HPV16 E1 fragment
<400> 42
   ggcgatctcg ctgtcgtcca cgatgtcgtt gtcgtaggcc cactgcacca tctggctcag 60
   ctcgaaggtg cagtcgttga agctgtgctg cagcacggtc tggcgctgga tccactcggg 120
   ggtgtcgcc 129
<210> 43
   <211> 129
   <212> DNA
   <213> Artificial Sequence
<220>
   <223> Codon-Optimized HPV16 E1 fragment
<400> 43
   gtggacgaca gcgagatcgc ctacaagtac gcccagctgg ccgacaccaa cagcaacgcc      60
   agcgccttcc tgaagagcaa cagccaggcc aagatcgtga aggactgcgc caccatgtgc     120
   cgccactac                                                             129
<210> 44
   <211> 129
   <212> DNA
   <213> Artificial Sequence
<220>
   <223> Codon-Optimized HPV16 E1 fragment
<400> 44
   gtagcgcagg aacatcacga tctgcttgcg gtcgccgccg tcgtccacgc ggtcgcagcg      60
   gtacttgatc cactggctca tgctcatctg cttcttctcg gcgcgcttgt agtggcggca     120
   catggtggc                                                             129
<210> 45
   <211> 129
   <212> DNA
   <213> Artificial Sequence
<220>
   <223> Codon-Optimized HPV16 E1 fragment
<400> 45
   cagatcgtga tgttcctgcg ctaccagggc gtggaattca tgagcttcct gaccgccctg      60
   aagcgcttcc tgcagggcat ccccaagaag aactgcatcc tgctgtacgg cgccgccaac     120
   accgacaag                                                             129
<210> 46
   <211> 130
   <212> DNA
   <213> Artificial Sequence
<220>
   <223> Codon-Optimized HPV16 E1 fragment
<400> 46
   gccgatcttg gcgtcggcca ggggctgcag ccagaagtgg ctcttgctgt tcacgaagca 60
   gatcacgctg ccctgcagga acttcatcag gctcatgccg aacaggctct tgtcggtgtt 120
   ggcggcgccg 130
<210> 47
   <211> 129
   <212> DNA
   <213> Artificial Sequence
<220>
   <223> Codon-Optimized HPV16 E1 fragment
<400> 47
   ctggccgacg ccaagatcgg catgctggac gacgccaccg tgccctgctg gaactacatc 60
   gacgacaacc tgcgcaacgc cctggacggc aacctggtga gcatggacgt gaagcaccgc 120
   cccctggtg 129
<210> 48
   <211> 132
   <212> DNA
   <213> Artificial Sequence
<220>
   <223> Codon-Optimized HPV16 E1 fragment
<400> 48
   gaactcgttg gggaaggtga acaccaccag gcggttgtgc aggtagggcc agcggctgtc 60
   ggtgccggcg ttgatgttgc tggtgatcag caggggaggg cacttcagct gcaccagggg 120
   gcggtgcttc ac 132
<210> 49
   <211> 126
   <212> DNA
   <213> Artificial Sequence
<220>
   <223> Codon-Optimized HPV16 E1 fragment
<400> 49
   gtgttcacct tccccaacga gttccccttc gacgagaacg gtaaccccgt gtacgagctg 60
   aacgacaaga actggaagag cttcttcagc cgcacctgga gccgcctgag cctgcacgag 120
   gacgag 126
<210> 50
   <211> 105
   <212> DNA
   <213> Artificial Sequence
<220>
   <223> Codon-Optimized HPV16 E1 fragment
<400> 50
   catgagagat ctttacaggg tgttggtgtt ctggccgctc acgcacttga aggtgggcag 60
   gctgtcgccg tcgttctcct tgtcctcgtc ctcgtgcagg ctcag 105
<210> 51
   <211> 23
   <212> DNA
   <213> Artificial Sequence
<220>
   <223> Codon-Optimized HPV16 E1 fragment
<400> 51
   gcctgaaggc catctgcatc gag 23
<210> 52
   <211> 23
   <212> DNA
   <213> Artificial Sequence
<220>
   <223> Codon-Optimized HPV16 E1 fragment
<400> 52
   ctcgatgcag atggccttca ggc 23
<210> 53
   <211> 21
   <212> DNA
   <213> Artificial Sequence
<220>
   <223> Codon-Optimized HPV16 E1 fragment
<400> 53
   gagctggtgc gccccttcaa g 21
<210> 54
   <211> 21
   <212> DNA
   <213> Artificial Sequence
<220>
   <223> Codon-Optimized HPV16 E1 fragment
<400> 54
   cttgaagggg cgcaccagct c 21
<210> 55
   <211> 21
   <212> DNA
   <213> Artificial Sequence
<220>
   <223> Codon-Optimized HPV16 E1 fragment
<400> 55
   ctgctgtgcg tgagccccat g 21
<210> 56
   <211> 21
   <212> DNA
   <213> Artificial Sequence
<220>
   <223> Codon-Optimized HPV16 E1 fragment
<400> 56
   catggggctc acgcacagca g 21
<210> 57
   <211> 21
   <212> DNA
   <213> Artificial Sequence
<220>
   <223> Codon-Optimized HPV16 E1 fragment
<400> 57
   gccaccatgt gccgccacta c 21
<210> 58
   <211> 21
   <212> DNA
   <213> Artificial Sequence
<220>
   <223> Codon-Optimized HPV16 E1 fragment
<400> 58
   gtagtggcgg cacatggtgg c 21
<210> 59
   <211> 21
   <212> DNA
   <213> Artificial Sequence
<220>
   <223> Codon-Optimized HPV16 E1 fragment
   <400> 59
   ctggccgacg ccaagatcgg c 21
<210> 60
   <211> 21
   <212> DNA
   <213> Artificial Sequence
<220>
   <223> Codon-Optimized HPV16 E1 fragment
<400> 60
   gccgatcttg gcgtcggcca g 21
<210> 61
   <211> 24
   <212> DNA
   <213> Artificial Sequence
<220>
   <223> Codon-Optimized HPV16 E1 fragment
<400> 61
   gtgttcacct tccccaacga gttc 24
<210> 62
   <211> 24
   <212> DNA
   <213> Artificial Sequence
<220>
   <223> Codon-Optimized HPV16 E1 fragment
<400> 62
   gaactcgttg gggaaggtga acac 24
<210> 63
   <211> 25
   <212> DNA
   <213> Artificial Sequence
<220>
   <223> Codon-Optimized HPV16 E1 fragment
<400> 63
   catgagagat ctttacaggg tgttg 25
<210> 64
   <211> 38
   <212> DNA
   <213> Artificial Sequence
<220>
   <223> Codon-Optimized HPV16 E1 fragment
<400> 64
   catctcagat ctgccaccat ggccgacccc gccggcac 38
<210> 65
   <211> 99
   <212> DNA
   <213> Artificial Sequence
<220>
   <223> Codon-Optimized HPV16 E2 fragment
<400> 65
   atggagaccc tgtgccagcg cctgaacgtg tgccaggaca agatcctgac ccactacgag 60
   aacgacagca ccgacctgcg cgaccacatc gactactgg 99
<210> 66
   <211> 104
   <212> DNA
   <213> Artificial Sequence
<220>
   <223> Codon-Optimized HPV16 E2 fragment
<400> 66
   ccaccaggtg gtgcccaccc tggccgtgag caagaacaag gccctgcagg ccgccgagct 60
   gcagctgacc ctggagacga tctacaacag ccagtacagc aacg 104
<210> 67
   <211> 108
   <212> DNA
   <213> Artificial Sequence
<220>
   <223> Codon-Optimized HPV16 E2 fragment
<400> 67
   ccggctgcat caagaagcac ggctacaccg tggaggtgca gttcgacggc gacatctgca 60
   acaccatgca ctacaccaac tggacccaca tttacatctg tgaggagg 108
<210> 68
   <211> 104
   <212> DNA
   <213> Artificial Sequence
<220>
   <223> Codon-Optimized HPV16 E2 fragment
<400> 68
   cgtgcacgag gggatccgca cctacttcgt gcagttcaag gacgacgccg agaagtacag 60
   caagaacaag gtgtgggagg tgcacgccgg aggccaggtg atcc 104
<210> 69
   <211> 110
   <212> DNA
   <213> Artificial Sequence
<220>
   <223> Codon-Optimized HPV16 E2 fragment
<400> 69
   ggccaaccac agcgccgcca cccacaccaa ggccgtggcc ctgggcaccg aggagaccca 60
   gaccacaatc cagcgccctc gcagcgagcc cgacaccggc aacccctgcc 110
<210> 70
   <211> 107
   <212> DNA
   <213> Artificial Sequence
<220>
   <223> Codon-Optimized HPV16 E2 fragment
<400> 70
   gccacaaggg ccggatcaac tgcaacagca acaccacccc tatcgtgcac ctgaagggcg 60
   acgccaacac cctgaagtgc ctgcggtacc gcttcaagaa gcactgc 107
<210> 71
   <211> 113
   <212> DNA
   <213> Artificial Sequence
<220>
   <223> Codon-Optimized HPV16 E2 fragment
<400> 71
   ccagggtggg caccacctgg tggttgatgt gcttgaagcc catctcgcgg gccttgtagt 60
   agatggcgca ggccaggcgc atgtgcttcc agtagtcgat gtggtcgcgc agg 113
<210> 72
   <211> 101
   <212> DNA
   <213> Artificial Sequence
<220>
   <223> Codon-Optimized HPV16 E2 fragment
<400> 72
   gccgtgcttc ttgatgcagc cggtaggggc ggtcaggtac acctccaggc tcacgtcctg 60
   cagggtccac ttctcgttgc tgtactggct gttgtagatc g 101
<210> 73
   <211> 98
   <212> DNA
   <213> Artificial Sequence
<220>
   <223> Codon-Optimized HPV16 E2 fragment
<400> 73
   ggtgcggatc ccctcgtgca cgtagtacag gccgtagtag tccacctggc cctccaccac 60
   ggtcacgctg gcctcctcac agatgtaaat gtgggtcc 98
<210> 74
   <211> 110
   <212> DNA
   <213> Artificial Sequence
<220>
   <223> Codon-Optimized HPV16 E2 fragment
<400> 74
   gggtggcggc gctgtggttg gccaggtgct ggcggatcgt ctcggggctg ctcacctcgt 60
   tgctgctgaa cacgctggtg gggcacagga tcacctggcc tccggcgtgc 110
<210> 75
   <211> 111
   <212> DNA
   <213> Artificial Sequence
<220>
   <223> Codon-Optimized HPV16 E2 fragment
<400> 75
   gcagttgatc cggcccttgt ggctgctgtt gaaggcggtc aggatagggg cgctgtcgac 60
   gctgtcgcgg tgcagcagct tggtggtgtg gcaggggttg ccggtgtcgg g 111
<210> 76
   <211> 110
   <212> DNA
   <213> Artificial Sequence
<220>
   <223> Codon-Optimized HPV16 E2 fragment
<400> 76
   cgtaggtcag ggtcacgata gcgctcttgt gcttcacgtt gtggccggtc cagtgccagg 60
   tgctgctcac ggcggtgtac agcttgcagt gcttcttgaa gcggtaccgc 110
<210> 77
   <211> 111
   <212> DNA
   <213> Artificial Sequence
<220>
   <223> Codon-Optimized HPV16 E2 fragment
<400> 77
   tttagatgct catgaagccg gtgctcacgg tgatggtctt ggggatcttc acctggctca 60
   ggaactggtc gcgctgccac tcgctgtcgt aggtcagggt cacgatagcg c 111
<210> 78
   <211> 50
   <212> DNA
   <213> Artificial Sequence
<220>
   <223> Codon-Optimized HPV16 E2 fragment
<400> 78
   cgagctgata tcgaattcag atctgccacc atggagaccc tgtgccagcg 50
<210> 79
   <211> 45
   <212> DNA
   <213> Artificial Sequence
<220>
   <223> Codon-Optimized HPV16 E2 fragment
<400> 79
   ggttgcagat ctagactcga gtttagatgc tcatgaagcc ggtgc 45
<210> 80
   <211> 21
   <212> DNA
   <213> Artificial Sequence
<220>
   <223> Codon-Optimized HPV16 E2 fragment
<400> 80
   ccggctgcat caagaagcac g 21
<210> 81
   <211> 19
   <212> DNA
   <213> Artificial Sequence
<220>
   <223> Codon-Optimized HPV16 E2 fragment
<400> 81
   ggccaaccac agcgccgcc 19
<210> 82
   <211> 21
   <212> DNA
   <213> Artificial Sequence
<220>
   <223> Codon-Optimized HPV16 E2 fragment
<400> 82
   gccgtgcttc ttgatgcagc c 21
<210> 83
   <211> 17
   <212> DNA
   <213> Artificial Sequence
<220>
   <223> Codon-Optimized HPV16 E2 fragment
<400> 83
   gggtggcggc gctgtgg 17
<210> 84
   <211> 22
   <212> DNA
   <213> Artificial Sequence
<220>
   <223> Codon-Optimized HPV16 E2 fragment
<400> 84
   cgtaggtcag ggtcacgata gc 22
<210> 85
   <211> 109
   <212> DNA
   <213> Artificial Sequence
<220>
   <223> Codon-Optimized HPV16 E7 fragment
<400> 85
   ggccggagat ctgatatcga attcgccacc atgcacggcg acacccccac cctgcacgag 60
   tacatgctgg acctgcagcc cgagaccacc gacctgtacg gctacggcc 109
<210> 86
   <211> 106
   <212> DNA
   <213> Artificial Sequence
<220>
   <223> Codon-Optimized HPV16 E7 fragment
<400> 86
   gccgagcccg accgcgccca ctacaacatc gtgaccttct gctgcaagtg cgacagcacc 60
   ctgcgcctgt gcgtgcagag cacccacgtc gacatccgca ccctgg 106
<210> 87
   <211> 96
   <212> DNA
   <213> Artificial Sequence
<220>
   <223> Codon-Optimized HPV16 E7 fragment
<400> 87
   gggcgcggtc gggctcggcc tggccggcgg ggccgtcgat ctcgtcctct tcctcgctgc 60
   tgtcgttcag ctggccgtag ccgtacaggt cggtgg 96
<210> 88
   <211> 106
   <212> DNA
   <213> Artificial Sequence
<220>
   <223> Codon-Optimized HPV16 E7 fragment
<400> 88
   ccgcggcaga tctagactcg agtttagggc ttctggctgc agattgggca cacgattccc 60
   agggtgccca tcagcaggtc ctccagggtg cggatgtcga cgtggg 106
<210> 89
   <211> 25
   <212> DNA
   <213> Artificial Sequence
<220>
   <223> Codon-Optimized HPV16 E7 fragment
<400> 89
   ggccggagat ctgatatcga attcg 25
<210> 90
   <211> 20
   <212> DNA
   <213> Artificial Sequence
<220>
   <223> Codon-Optimized HPV16 E7 fragment
<400> 90
   ccgcggcaga tctagactcg 20
<210> 91
   <211> 105
   <212> DNA
   <213> Artificial Sequence
<220>
   <223> Codon-Optimized HPV6a E7 fragment
<400> 91
   gtcacagatc tgatatcgaa ttccaccatg cacggccgcc acgtgaccct gaaggacatc 60
   gtgctggacc tgcagcctcc cgaccccgtg ggcctgcact gctac 105
<210> 92
   <211> 105
   <212> DNA
   <213> Artificial Sequence
<220>
   <223> Codon-Optimized HPV6a E7 fragment
<400> 92
   ctggaagtgc tgcttcaggg gctggctgtc ctggccgtcc acctcgtcca cctcgtcctc 60
   gctgctgtcc accagctgct cgtagcagtg caggcccacg gggtc 105
<210> 93
   <211> 107
   <212> DNA
   <213> Artificial Sequence
<220>
   <223> Codon-Optimized HPV6a E7 fragment
<400> 93
   ccagcccctg aagcagcact tccagatcgt gacctgctgc tgcggctgcg acagcaacgt 60
   gcgcctggtg gtgcagtgca ccgagaccga catccgcgag gtgcagc 107
<210> 94
   <211> 102
   <212> DNA
   <213> Artificial Sequence
<220>
   <223> Codon-Optimized HPV6a E7 fragment
<400> 94
   cagtcagatc tagagatatc tttaggtctt gggagcgcag atggggcaca cgatgttcag 60
   ggtacccagc aggagctgct gcacctcgcg gatgtcggtc tc 102
<210> 95
   <211> 24
   <212> DNA
   <213> Artificial Sequence
<220>
   <223> PCR Primer
<400> 95
   gtcacagatc tgatatcgaa ttcc 24
<210> 96
   <211> 26
   <212> DNA
   <213> Artificial Sequence
<220>
   <223> PCR Primer
<400> 96
   cagtcagatc tagagatatc tttagg 26
<210> 97
   <211> 109
   <212> DNA
   <213> Artificial Sequence
<220>
   <223> Codon-Optimized HPV18 E7 fragment
<400> 97
   gtcacagatc tgatatcgaa ttccaccatg cacggcccca aggccaccct gcaggacatc 60
   gtgctgcacc tggagcccca gaacgagatc cccgtggacc tgctgtgcc 109
<210> 98
   <211> 111
   <212> DNA
   <213> Artificial Sequence
<220>
   <223> Codon-Optimized HPV18 E7 fragment
<400> 98
   gggctcggcc ctgcgagcgg gcaggtgctg gtggttcacg ccgtcgatct cgtcgttctc 60
   ctcctcgctg tcgctcagct gctcgtggca cagcaggtcc acggggatct c 111
<210> 99
   <211> 108
   <212> DNA
   <213> Artificial Sequence
<220>
   <223> Codon-Optimized HPV18 E7 fragment
<400> 99
   gcccgctcgc agggccgagc cccagcgcca caccatgctg tgcatgtgct gcaagtgcga 60
   ggcccgcatc gagctggtgg tggagagcag cgctgacgac ctgcgcgc 108
<210> 100
   <211> 109
   <212> DNA
   <213> Artificial Sequence
<220>
   <223> Codon-Optimized HPV18 E7 fragment
<400> 100
   cagtcagatc tagagatatc tttactgctg gctggcgcac caggggcaca cgaagctcag 60
   ggtgttcagg aacagctgct ggaaggcgcg caggtcgtca gcgctgctc 109
<210> 101
   <211> 26
   <212> DNA
   <213> Artificial Sequence
<220>
   <223> PCR Fragment
<400> 101
   gtcacagatc tgatatcgaa ttccac 26
<210> 102
   <211> 27
   <212> DNA
   <213> Artificial Sequence
<220>
   <223> PCR Fragment
<400> 102
   cagtcagatc tagagatatc tttactg 27
<210> 103
   <211> 90
   <212> DNA
   <213> Artificial Sequence
<220>
   <223> Codon-Optimized HPV6 E2 fragment
<400> 103
   gaattcagat ctgatatcac catggaggcc atcgccaagc gcctggacgc ctgccaggag 60
   cagctgctgg agctgtacga ggagaacagc 90
<210> 104
   <211> 92
   <212> DNA
   <213> Artificial Sequence
<220>
   <223> Codon-Optimized HPV6 E2 fragment
<400> 104
   ccttgtacag cagcacgctc tcgtggcgca tgcacttcca gtgcagcacg tgcttgtgca      60
   ggtcggtgct gttctcctcg tacagctcca gc                                    92
<210> 105
   <211> 96
   <212> DNA
   <213> Artificial Sequence
<220>
   <223> Codon-Optimized HPV6 E2 fragment
<400> 105
   ccacgagagc gtgctgctgt acaaggccaa gcagatgggc ctgagccaca tcggcatgca 60
   ggtggtgcct cctctgaagg tgagcgaggc caaggg 96
<210> 106
   <211> 103
   <212> DNA
   <213> Artificial Sequence
<220>
   <223> Codon-Optimized HPV6 E2 fragment
<400> 106
   gcagggtcca gggctccatg ctgtactcgg tgcgcagcag gctctcgagg tgcatctgca 60
   tctcgatggc gttgtggccc ttggcctcgc tcaccttcag agg 103
<210> 107
   <211> 98
   <212> DNA
   <213> Artificial Sequence
<220>
   <223> Codon-Optimized HPV6 E2 fragment
<400> 107
   cgagtacagc atggagccct ggaccctgca ggagaccagc tacgagatgt ggcagacccc 60
   tcccaagcgc tgcttcaaga agcgcggcaa gaccgtgg 98
<210> 108
   <211> 102
   <212> DNA
   <213> Artificial Sequence
<220>
   <223> Codon-Optimized HPV6 E2 fragment
<400> 108
   cgttgtcctg cacgtacacg tcggtccaca ccacgtagtc catggtgttg ttggcgcagc 60
   cgtcgaactt cacctccacg gtcttgccgc gcttcttgaa gc 102
<210> 109
   <211> 102
   <212> DNA
   <213> Artificial Sequence
<220>
   <223> Codon-Optimized HPV6 E2 fragment
<400> 109
   ccgacgtgta cgtgcaggac aacgacacct gggtgaaggt gcacagcatg gtggacgcca 60
   agggcatcta ctacacctgt ggccagttca agacctacta cg 102
<210> 110
   <211> 92
   <212> DNA
   <213> Artificial Sequence
<220>
   <223> Codon-Optimized HPV6 E2 fragment
<400> 110
   gctgccgtag cacacctccc agtgcttggt gctgccgtac ttctcggcct ccttcacgaa 60
   gttcacgtag taggtcttga actggccaca gg 92
<210> 111
   <211> 94
   <212> DNA
   <213> Artificial Sequence
<220>
   <223> Codon-Optimized HPV6 E2 fragment
<400> 111
   gcactgggag gtgtgctacg gcagcaccgt gatctgcagc cccgctagcg tgagcagcac 60
   cacccaggag gtgagcatcc ccgagagcac cacc 94
<210> 112
   <211> 97
   <212> DNA
   <213> Artificial Sequence
<220>
   <223> Codon-Optimized HPV6 E2 fragment
<400> 112
   gcgaggaggg gtctgcacgg cgtcctcctt ggtgctgctg ctcaccaggg tgctggtctg 60
   ggcgggagtg taggtggtgc tctcggggat gctcacc 97
<210> 113
   <211> 97
   <212> DNA
   <213> Artificial Sequence
<220>
   <223> Codon-Optimized HPV6 E2 fragment
<400> 113
   ggacgccgtg cagacccctc ctcgcaagcg cgcccgcggc gtgcagcaga gcccctgcaa 60
   cgccctgtgc gtggcccaca tcggccccgt ggacagc 97
<210> 114
   <211> 94
   <212> DNA
   <213> Artificial Sequence
<220>
   <223> Codon-Optimized HPV6 E2 fragment
<400> 114 <210> 115
   <211> 95
   <212> DNA
   <213> Artificial Sequence
<220>
   <223> Codon-Optimized HPV6 E2 fragment
<400> 115
<210> 116
   <211> 96
   <212> DNA
   <213> Artificial Sequence
<220>
   <223> Codon-Optimized HPV6 E2 fragment
<400> 116
   cgtgcttgtg gggagccttg ctgctggccc agtgccaggt gctgctgatc aggtcgaaca 60
   ggtggcggtg gcgatcgttc aggcggtagc ggaagc 96
<210> 117
   <211> 95
   <212> DNA
   <213> Artificial Sequence
<220>
   <223> Codon-Optimized HPV6 E2 fragment
<400> 117
   gcagcaaggc tccccacaag cacgccatcg tgaccgtgac ctacgacagc gaggagcagc 60
   gccagcagtt cctggacgtg gtgaagatcc ctccc 95
<210> 118
   <211> 96
   <212> DNA
   <213> Artificial Sequence
<220>
   <223> Codon-Optimized HPV6 E2 fragment
<400> 118
   ctcgagagat ctcccgggtc tagagcttac agcaggtgca ggctcatgaa gcccagcttg 60
   tggctgatgg tgggagggat cttcaccacg tccagg 96
<210> 119
   <211> 25
   <212> DNA
   <213> Artificial Sequence
<220>
   <223> Codon-Optimized HPV6 E2 fragment
<400> 119
   gaattcagat ctgatatcac catgg 25
<210> 120
   <211> 21
   <212> DNA
   <213> Artificial Sequence
<220>
   <223> Codon-Optimized HPV6 E2 fragment
<400> 120
   gcagggtcca gggctccatg c 21
<210> 121
   <211> 25
   <212> DNA
   <213> Artificial Sequence
<220>
   <223> Codon-Optimized HPV6 E2 fragment
<400> 121
   cgagtacagc atggagccct ggacc 25
<210> 122
   <211> 25
   <212> DNA
   <213> Artificial Sequence
<220>
   <223> Codon-Optimized HPV6 E2 fragment
<400> 122
   gctgccgtag cacacctccc agtgc 25
<210> 123
   <211> 21
   <212> DNA
   <213> Artificial Sequence
<220>
   <223> Codon-Optimized HPV6 E2 fragment
<400> 123
   gcactgggag gtgtgctacg g 21
<210> 124
   <211> 23
   <212> DNA
   <213> Artificial Sequence
<220>
   <223> Codon-Optimized HPV6 E2 fragment
<400> 124
   ggcgctgctg ttgctgttgt tgc 23
<210> 125
   <211> 22
   <212> DNA
   <213> Artificial Sequence
<220>
   <223> Codon-Optimized HPV6 E2 fragment
<400> 125
   ccgcaacaac agcaacagca gc 22
<210> 126
   <211> 26
   <212> DNA
   <213> Artificial Sequence
<220>
   <223> Codon-Optimized HPV6 E2 fragment
<400> 126
   ctcgagagat ctcccgggtc tagagc 26
<210> 127
   <211> 97
   <212> DNA
   <213> Artificial Sequence
<220>
   <223> Codon-Optimized HPV18 E2 fragment
<400> 127
   gaattcagat ctgatatcac catgcagact cccaaggaga ccctgagcga gcgcctgagc 60
   gccctgcagg acaagatcat cgaccactac gagaacg 97
<210> 128
   <211> 98
   <212> DNA
   <213> Artificial Sequence
<220>
   <223> Codon-Optimized HPV18 E2 fragment
<400> 128
   cgaagaagat ggcgttctcc cagcggatca gctgccagta ctggatctgg ctgtcgatgt 60
   ccttgctgtc gttctcgtag tggtcgatga tcttgtcc 98
<210> 129
   <211> 94
   <212> DNA
   <213> Artificial Sequence
<220>
   <223> Codon-Optimized HPV18 E2 fragment
<400> 129
   ccgctgggag aacgccatct tcttcgccgc tcgcgagcac gggatccaga ccctgaacca 60
   ccaggtggtg cccgcctaca acatcagcaa gagc 94
<210> 130
   <211> 94
   <212> DNA
   <213> Artificial Sequence
<220>
   <223> Codon-Optimized HPV18 E2 fragment
<400> 130
   cctcggtctt gtaggcgctc tgggccaggc cctgcagggc catctgcagc tcgatggcct 60
   tgtgggcctt gctcttgctg atgttgtagg cggg 94
<210> 131
   <211> 91
   <212> DNA
   <213> Artificial Sequence
<220>
   <223> Codon-Optimized HPV18 E2 fragment
<400> 131
   cccagagcgc ctacaagacc gaggactgga ccctgcagga cacctgcgag gagctgtgga 60
   acaccgagcc cacccactgc ttcaagaagg g 91
<210> 132
   <211> 94
   <212> DNA
   <213> Artificial Sequence
<220>
   <223> Codon-Optimized HPV18 E2 fragment
<400> 132
   gctgtcccag gccacgtagt tcatgcagtt gtccttgttg ccgtcgaagt acacctgcac 60
   ggtctggcct cccttcttga agcagtgggt gggc 94
<210> 133
   <211> 90
   <212> DNA
   <213> Artificial Sequence
<220>
   <223> Codon-Optimized HPV18 E2 fragment
<400> 133
   gcatgaacta cgtggcctgg gacagcgtgt actacatgac cgacgccggc acctgggaca 60
   agaccgccac ctgcgtgagc caccgcggcc 90
<210> 134
   <211> 92
   <212> DNA
   <213> Artificial Sequence
<220>
   <223> Codon-Optimized HPV18 E2 fragment
<400> 134
   ccgtacttct cgcactcgct cttgaactcg atgtagaagg tgttgtagcc ctccttcacg 60
   tagtacaggc cgcggtggct cacgcaggtg gc 92
<210> 135
   <211> 94
   <212> DNA
   <213> Artificial Sequence
<220>
   <223> Codon-Optimized HPV18 E2 fragment
<400> 135
   cgagttcaag agcgagtgcg agaagtacgg caacaccggc acctgggagg tgcacttcgg 60
   caacaacgtg atcgactgca acgacagcat gtgc 94
<210> 136
   <211> 100
   <212> DNA
   <213> Artificial Sequence
<220>
   <223> Codon-Optimized HPV18 E2 fragment
<400> 136
   gctgtagggg ctgggagtgt gctgcagctg cttcaccagc tgggtggcgc tcacggtgtc 60
   gtcgctggtg ctgcacatgc tgtcgttgca gtcgatcacg 100
<210> 137
   <211> 93
   <212> DNA
   <213> Artificial Sequence
<220>
   <223> Codon-Optimized HPV18 E2 fragment
<400> 137
   gcacactccc agcccctaca gcagcaccgt gagcgtgggc accgccaaga cctacggcca 60
   gaccagcgcc gccactcgcc ctggccactg cgg 93
<210> 138
   <211> 96
   <212> DNA
   <213> Artificial Sequence
<220>
   <223> Codon-Optimized HPV18 E2 fragment
<400> 138
   gcttgttgtt gccggtggcg gtggcggcgc ccagcagagg gttcacgggc ccgcagtgct 60
   gcttctcggc caggccgcag tggccagggc gagtgg 96
<210> 139
   <211> 97
   <212> DNA
   <213> Artificial Sequence
<220>
   <223> Codon-Optimized HPV18 E2 fragment
<400> 139
   gccaccgcca ccggcaacaa caagcgccgc aagctgtgca gcggcaacac cactcccatc 60
   atccacctga agggcgaccg caacagcctg aagtgcc 97
<210> 140
   <211> 97
   <212> DNA
   <213> Artificial Sequence
<220>
   <223> Codon-Optimized HPV18 E2 fragment
<400> 140
   ggcgccggtc cagtgccagg tgctgctgat gtcgcggtag tggtcgctgt gcttgcgcag 60
   gcggtaccgc aggcacttca ggctgttgcg gtcgccc 97
<210> 141
   <211> 99
   <212> DNA
   <213> Artificial Sequence
<220>
   <223> Codon-Optimized HPV18 E2 fragment
<400> 141
   gcacctggca ctggaccggc gccgggaacg agaagaccgg catcctgacc gtgacctacc 60
   acagcgagac ccagcgcacc aagttcctga acaccgtgg 99
<210> 142
   <211> 98
   <212> DNA
   <213> Artificial Sequence
<220>
   <223> Codon-Optimized HPV18 E2 fragment
<400> 142
   ctcgagagat ctcccgggtc tagagcttac atggtcatgt agcccaccag gatctgcacg 60
   ctgtcgggga tggccacggt gttcaggaac ttggtgcg 98
<210> 143
   <211> 25
   <212> DNA
   <213> Artificial Sequence
<220>
   <223> Codon-Optimized HPV18 E2 fragment
<400> 143
   gaattcagat ctgatatcac catgc 25
<210> 144
   <211> 23
   <212> DNA
   <213> Artificial Sequence
<220>
   <223> Codon-Optimized HPV18 E2 fragment
<400> 144
   cctcggtctt gtaggcgctc tgg 23
<210> 145
   <211> 21
   <212> DNA
   <213> Artificial Sequence
<220>
   <223> Codon-Optimized HPV18 E2 fragment
<400> 145
   cccagagcgc ctacaagacc g 21
<210> 146
   <211> 21
   <212> DNA
   <213> Artificial Sequence
<220>
   <223> Codon-Optimized HPV18 E2 fragment
<400> 146
   ccgtacttct cgcactcgct c 21
<210> 147
   <211> 20
   <212> DNA
   <213> Artificial Sequence
<220>
   <223> Codon-Optimized HPV18 E2 fragment
<400> 147
   cgagttcaag agcgagtgcg 20
<210> 148
   <211> 21
   <212> DNA
   <213> Artificial Sequence
<220>
   <223> Codon-Optimized HPV18 E2 fragment
<400> 148
   gcttgttgtt gccggtggcg g 21
<210> 149
   <211> 25
   <212> DNA
   <213> Artificial Sequence
<220>
   <223> Codon-Optimized HPV18 E2 fragment
<400> 149
   gccaccgcca ccggcaacaa caagc 25
<210> 150
   <211> 26
   <212> DNA
   <213> Artificial Sequence
<220>
   <223> Codon-Optimized HPV18 E2 fragment
<400> 150
   ctcgagagat ctcccgggtc tagagc                                                26